# EUROPÄISCHE PATENTSCHRIFT

(11) **EP 1 414 803 B1**
(45) Veröffentlichungstag und Bekanntmachung des Hinweises auf die Patenterteilung: **12.12.2012**
(21) Anmeldenummer: 02753080.7
(22) Anmeldetag: 12.07.2002
(51) Int. Cl.: C07D 231/16, A01N 43/56

(54) **PYRAZOLYLKARBOXANILIDE ALS FUNGIZIDE**
PYRAZOLYLCARBOXANILIDES AS FUNGICIDES
CARBOXANILIDES DE PYRAZOLYLE UTILISES COMME FONGICIDES

(30) Priorität: 25.07.2001 DE 10136065
(43) Veröffentlichungstag der Anmeldung: 06.05.2004
(73) Patentinhaber: Bayer CropScience AG, 40789 Monheim (DE)
(72) Erfinder: ELBE, Hans-Ludwig, 42329 Wuppertal (DE); RIECK, Heiko, 69110 Ste. Foy lès Lyon (FR); DUNKEL, Ralf, 40789 Monheim (DE); ZHU-OHLBACH, Qin, 40219 Düsseldorf (DE); MAULER-MACHNIK, Astrid, 42799 Leichlingen (DE); WACHENDORFF-NEUMANN, Ulrike, 56566 Neuwied (DE); KUCK, Karl-Heinz, 40764 Langenfeld (DE)
(86) Internationale Anmeldenummer: PCT/EP2002/007779
(87) Internationale Veröffentlichungsnummer: WO 2003/010149

(56) Entgegenhaltungen:
- EP-A- 0 545 099
- WO-A-02/08195
- DATABASE CA [Online] CHEMICAL ABSTRACTS SERVICE, COLUMBUS, OHIO, US; NISHIDA, SUMIO ET AL: "Preparation of 5-fluoro-pyrazole-4-carboxamides as agrochemical microbicides" retrieved from STN Database accession no. 109:231009 XP002218232 in der Anmeldung erwähnt & JP 63 048269 A (SUMITOMO CHEMICAL CO., LTD., JAPAN) 29. Februar 1988 (1988-02-29)
- DATABASE CA [Online] CHEMICAL ABSTRACTS SERVICE, COLUMBUS, OHIO, US; NISHIDA, SUMIO ET AL: "Preparation of 3'-isopropoxy-2'-methylanilides as fungicides" retrieved from STN Database accession no. 109:124408 XP002218233 & JP 62 249975 A (SUMITOMO CHEMICAL CO., LTD., JAPAN) 30. Oktober 1987 (1987-10-30)

## Beschreibung

Die vorliegende Erfindung betrifft neue Pyrazolylcarboxanilide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vergleiche WO 93-11117, EP-A 0 545 099, EP-A 0 589 301, WO 99/09013, DE 198 40 322, EP-A 0 824 099, JP 63048269). So lassen sich 1,3-Dimethyl-5-fluorpy-razol-4-carbonsäure-(2-cyclohexyl)-anilid, 1,3-Dimethyl-pyrazol-4-carbonsäure-(2-phenyl)-anilid und 1,3-Dimethyl-pyrazol-4-carbonsäure-[2-(2-fluor-phenyl)]-anilid zur Bekämpfung von Pilzen einsetzen. Die Wirksamkeit dieser Stoffe ist gut, lässt aber bei niedrigen Aufwandmengen in manchen Fällen zu wünschen übrig.

Es wurden nun neue Pyrazolylcarboxanilide der Formel (I) 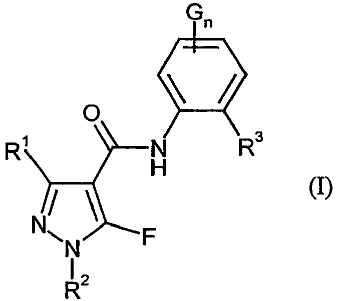 gefunden, in welcher
- R¹: für Wasserstoff, Cyano, Halogen, Nitro, C₁-C₄-Alkyl, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkylthio, C₁-C₄-Halogenalkylthio mit 1 bis 5 Halogenatomen oder Aminocarbonyl-C₁-C₄-alkyl steht,
- R²: für Wasserstoff, C₁-C₄-Alkyl, C₁-C₆-Halogenalkyl mit 1 bis 5 Halogenatomen, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Halogenatomen, C₁-C₄-Alkoxy-C₁-C₄-alkyl oder C₁-C₄-Halogenalkoxy-C₁-C₄-alkyl mit 1 bis 5 Halogenatomen steht,
- G: für Halogen oder C₁-C₄-Alkyl steht,
- G: außerdem für C₅-C₆-Alkyl steht,
- R³: für unsubstituiertes C₅-C₂₀ Alkyl oder für einfach oder mehrfach, gleich oder verschieden durch Halogen und/oder C₃-C₆-Cycloalkyl substituiertes C₁-C₂₀-Alkyl oder für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann und
- n: für 0, 1 oder 2 steht.

Die erfindungsgemäßen Verbindungen können gegebenenfalls als Mischungen verschiedener möglicher isomerer Formen, insbesondere von Stereoisomeren, wie z.B. B- und Z-, threo- und erythro-, sowie optischen Isomeren, gegebenenfalls aber auch von Tautomeren vorliegen. Es werden sowohl die E- als auch die Z-Isomeren, wie auch die threo- und eythro-, sowie die optischen Isomeren, beliebige Mischungen dieser Isomeren, sowie die möglichen tautomeren Formen beansprucht.

Weiterhin wurde gefunden, dass man Pyrazolylcarboxanilide der Formel (I) erhält, indem man
a) Carbonsäure-Derivate der Formel (II) 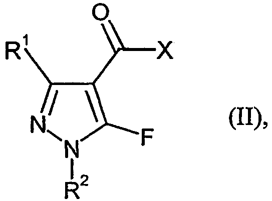 in welcher
   - R¹ und R²: die oben angegebenen Bedeutungen haben und
   - X: für Halogen steht,
   mit einem Anilinderivat der Formel (III) 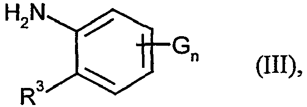 in welcher
   - G, R³ und n: die oben angegebenen Bedeutungen haben,
   gegebenenfalls in Gegenwart eines Säurebindemittels und gegebenenfalls in Gegenwart eines Verdünnungsmittels umsetzt, oder
b) Pyrazolylcarboxanilide der Formel (Ia) 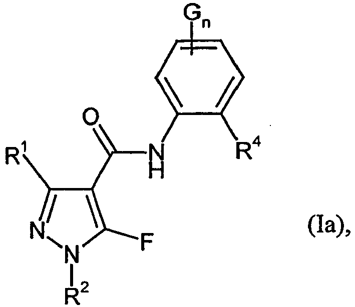 in welcher
   - R¹, R², G und n: die oben angegebenen Bedeutungen haben und
   - R⁴: für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart eines Katalysators hydriert, oder
c) Hydroxyalkylpyrazolylcarboxanilide der Formel (IV) 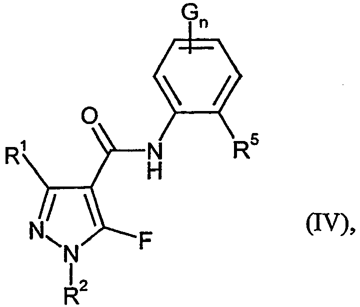 in welcher
   - R¹, R², G und n: die oben angegebenen Bedeutungen haben und
   - R⁵: für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart einer Säure dehydratisiert, oder
d) Halogenpyrazolylcarboxanilide der Formel (V) 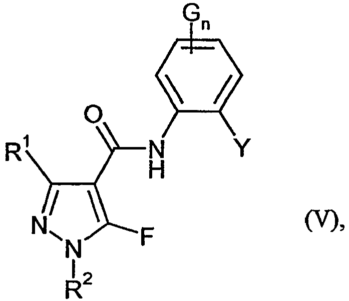 in welcher
   - R¹, R², G und n: die oben angegebenen Bedeutungen haben und
   - Y: für Brom oder Iod steht,
   mit einem Alkin der Formel (VI) 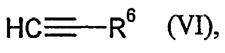 in welcher
   - R⁶: für gegebenenfalls einfach oder mehrfach, gleich oder verschieden durch Halogen und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
   oder einem Alken der Formel (VII) 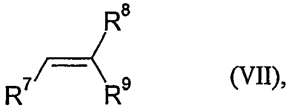 in welcher
   - R⁷, R⁸ und R⁹: unabhängig voneinander jeweils für Wasserstoff oder gegebenenfalls einfach oder mehrfach, gleich oder verschieden durch Halogen und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 20 nicht übersteigt,
   gegebenenfalls in Gegenwart eines Verdünnungsmittels, gegebenenfalls in Gegenwart eines Säurebindemittels und in Gegenwart eines oder mehrerer Katalysatoren umsetzt, oder
e) Ketone der Formel (VIII) 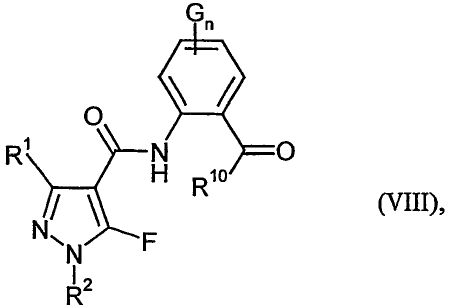 in welcher
   - R¹, R², G und n: die oben angegebenen Bedeutungen haben und
   - R¹⁰: für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   mit einer Phosphorverbindung der allgemeinen Formel (IX)

   R¹¹―Px (IX),

   in welcher
   - R¹¹: für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   - Px: für eine Gruppierung -P⁺(C₆H₅)₃ Cl⁻, -P⁺(C₆H₅)₃ Br, -P⁺(C₆H₅)₃I⁻, -P(=O)(OCH₃)₃ oder -P(=O)(OC₂H₅)₃ steht,
   gegebenenfalls in Gegenwart eines Verdünnungsmittels umsetzt.

Schließlich wurde gefunden, dass die neuen Pyrazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Pyrazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Pyrazolylcarboxanilide sind durch die Formel (I) allgemein definiert.
- R¹: für steht bevorzugt für Wasserstoff, Cyano, Halogen, Nitro, C₁-C₄-Alkyl, C₁-C₄-Halogenalkyl mit 1 bis 5 Fluor-, Chlor- und/oder Bromatomen, C₃-C₆-Cycloalkyl, C₁-C₄-Alkoxy, C₁-C₄-Halogenalkoxy mit 1 bis 5 Fluor-, Chlor-und/oder Bromatomen, C₁-C₄-Alkylthio, C₁-C₄-Halogenalkylthio mit 1 bis 5 Fluor-, Chlor- und/oder Bromatomen oder Aminocarbonyl-C₁-C₄-alkyl.
- R²: steht bevorzugt für Wasserstoff, C₁-C₄-Alkyl, C₁-C₆-Halogenalkyl mit 1 bis 5 Fluor-, Chlor- und/oder Bromatomen, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Fluor-, Chlor- und/oder Bromatomen, C₁-C₄-Alkoxy-C₁-C₄-alkyl oder C₁-C₄-Halogenalkoxy-C₁-C₄-alkyl mit 1 bis 5 Fluor-, Chlor- und/oder Bromatomen.
- G: steht bevorzugt für Halogen oder C₁-C₄-Alkyl.
- G: steht außerdem bevorzugt für C₅-C₆-Alkyl.
- R³: steht bevorzugt für unsubstituiertes C₆-C₁₃ Alkyl oder für einfach bis vierfach, gleich oder verschieden durch Fluor, Chlor, Brom und/oder C₃-C₆-Cycloalkyl substituiertes C₁-C₁₂-Alkyl oder für jeweils gegebenenfalls einfach bis vierfach, gleich oder verschieden durch Fluor, Chlor, Brom und/oder C₃-C₆-Cycloalkyl substituiertes C₂-C₁₂-Alkenyl oder C₂-C₁₂-Alkinyl, wobei der Cycloalkylteil seinerseits gegebenenfalls einfach bis vierfach, gleich oder verschieden durch Halogen und/oder C₁-C₄-Alkyl substituiert sein kann.
- n: steht bevorzugt für 0, 1 oder 2.
- R¹: steht besonders bevorzugt für Wasserstoff, Methyl, Ethyl, n- oder i-Propyl, n-, i-, s- oder t-Butyl, Trifluormethyl oder Trifluorethyl.
- R²: steht besonders bevorzugt für Wasserstoff, Methyl, Ethyl, n- oder i-Propyl, n-, i-, s- oder t-Butyl, Trifluormethyl oder Trifluorethyl.
- G: steht besonders bevorzugt für Fluor, Chlor oder Methyl.
- G: steht außerdem besonders bevorzugt für Ethyl oder t-Butyl.
- G: steht außerdem besonders bevorzugt für 2,4-Dimethylbutyl.
- R³: steht besonders bevorzugt für jeweils gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl, Octyl, Nonyl, Decyl, Ethenyl, Propenyl, Butenyl, Pentenyl, Hexenyl, Heptenyl, Octenyl, Nonenyl, Decenyl, Ethinyl, Propinyl, Butinyl, Pentinyl, Hexinyl, Heptinyl, Octinyl, Noninyl oder Decinyl.
- n: steht auch besonders bevorzugt für 0, 1 oder 2.

Eine ganz besonders bevorzugte Gruppe sind Verbindungen der Formel (I), in welcher
- R¹: für Methyl steht und
- R²: für Methyl steht.

Weiterhin ganz besonders bevorzugt sind Verbindungen der Formel (I), in welcher
- R³: für unsubstituiertes C₆-C₂₀ steht.

Weiterhin ganz besonders bevorzugt sind Verbindungen der Formel (I), in welcher
- n: für 0 steht.

Hervorgehoben sind Verbindungen der Formel (I), in welcher
- R¹: für Methyl steht,
- R²: für Methyl steht,
- R³: für 1,3-Dimethylbutyl steht,
- n: für 0 steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Mehrere Reste mit denselben Indizes wie beispielsweise n Reste G für n >1, können gleich oder verschieden sein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Verwendet man 5-Fluor-1,3-dimethyl-1H-pyrazol-4-carbonyl-chlorid und 2-(1-Methylhexyl)anilin als Ausgangsstoffe, so kann das erfindungsgemäße Verfahren a) durch das folgende Formelschema veranschaulicht werden:

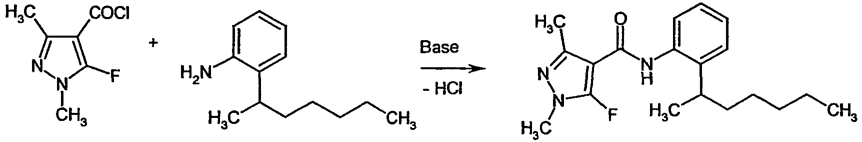

Die zur Durchführung des erfindungsgemäßen Verfahrens a) als Ausgangsstoffe benötigten Carbonsäure-Derivate sind durch die Formel (II) allgemein definiert. In dieser Formel (II) haben R¹ und R² bevorzugt bzw. besonderes bevorzugt diejenigen Bedeutungen, die bereits im Zusammenhang mit der Beschreibung der erfindungsgemäßen Verbindungen der Formel (I) als bevorzugt bzw. als besonders bevorzugt für R¹ und R² angegeben wurden. X steht für Halogen, bevorzugt für Chlor.

Die Carbonsäure-Derivate der Formel (II) sind bekannt und/oder lassen sich nach bekannten Verfahren herstellen (vgl. WO 93/11117, EP-A 0 545 099, EP-A 0 589 301 und EP-A 0 589 313).

Die zur Durchführung des erfindungsgemäßen Verfahrens a) als Ausgangsstoffe weiterhin benötigten Aniline sind durch die Formel (III) allgemein definiert. In dieser Formel (III) haben G, R³ und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für G, R³ und n angegeben wurden.

Die Anilin-Derivate der Formel (III) sind bekannt und/oder lassen sich nach bekannten Methoden herstellen (vergleiche z. B. Heterocycles (1989), 29(6), 1013-16; J. Med. Chem. (1996), 39(4), 892-903; Synthesis (1995), (6), 713-16; Synth. Commun. (1994), 24(2), 267-72; DE 2727416; Synthesis (1994), (2), 142-4; EP 0 824 099).

Verwendet man 5-Fluor-1,3-dimethyl-N-{2-[(1Z)-1-methyl-1-hexenyl]phenyl}-1H-pyrazol-4-carboxamid und Wasserstoff als Ausgangsstoffe sowie einen Katalysator, so kann der Verlauf des erfindungsgemäßen Verfahrens b) durch das folgende Formelschema veranschaulicht werden:

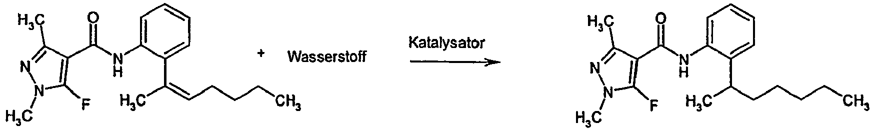

Die zur Durchführung des erfindungsgemäßen Verfahrens b) als Ausgangsstoffe benötigten Pyrazolylcarboxanilide sind durch die Formel (Ia) allgemein definiert. In dieser Formel (Ia) haben R¹, R², G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. als besonders bevorzugt für R¹, R², G und n angegeben wurden.
- R⁴: steht bevorzugt für jeweils gegebenenfalls einfach bis vierfach, gleich oder verschieden durch Fluor, Chlor und/oder C₃-C₆-Cycloalkyl substituiertes C₂-C₁₂-Alkenyl oder C₂-C₁₂-Alkinyl, wobei der Cycloalkylteil seinerseits gegebenenfalls einfach bis vierfach, gleich oder verschieden durch Halogen und/oder C₁-C₄-Alkyl substituiert sein kann.
- R⁴: steht besonders bevorzugt für jeweils gegebenenfalls einfach bis vierfach, gleich oder verschieden durch Fluor, Cyclopropyl, Difluorcyclopropyl, Cyclobutyl, Cyclopentyl oder Cyclohexyl substituiertes, jeweils geradkettiges oder verzweigtes, jeweils an beliebiger Stelle verknüpftes Ethenyl, Propenyl, Butenyl, Pentenyl, Hexenyl, Heptenyl, Octenyl, Nonenyl, Decenyl, Ethinyl, Propinyl, Butinyl, Pentinyl, Hexinyl, Heptinyl, Octinyl, Noninyl oder Decinyl.

Die Verbindungen der Formel (Ia) sind erfindungsgemäße Verbindungen und können nach Verfahren a), c), d) oder e) hergestellt werden.

Verwendet man 5-Fluor-N-[2-(1-hydroxy-1-methylhexyl)phenyl]-1,3-dimethyl-1H-pyrazol-4-carboxamid als Ausgangsstoff sowie eine Säure, so kann der Verlauf des erfindungsgemäßen Verfahrens c) durch das folgende Formelschema veranschaulicht werden:

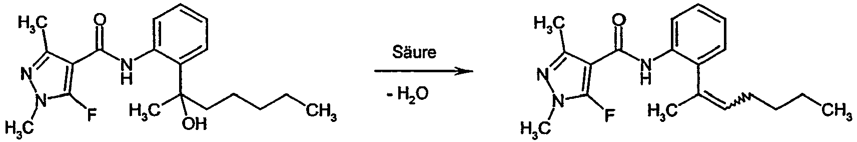

Die zur Durchführung des erfindungsgemäßen Verfahrens c) als Ausgangsstoffe benötigten Hydroxyalkylpyrazolylcarboxanilide sind durch die Formel (IV) allgemein definiert. In dieser Formel (IV) haben R¹, R², G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für R¹, R², G und n angegeben wurden.
- R⁵: steht bevorzugt für gegebenenfalls zusätzlich einfach bis vierfach, gleich oder verschieden durch Chlor, Fluor, Brom und/oder C₃-C₆-Cycloalkyl substituiertes C₂-C₁₂- Hydroxyalkyl, wobei der Cycloalkylteil seinerseits gegebenenfalls durch Halogen und/oder C₁-C₄-Alkyl substituiert sein kann.
- R⁵: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Hydroxyethyl, Hydroxypropyl, Hydroxybutyl, Hydroxypentyl, Hydroxyhexyl, Hydroxyheptyl, Hydroxyoctyl, Hydroxynonyl oder Hydroxydecyl.

Die Verbindungen der Formel (IV) sind noch nicht bekannt und als neue Verbindungen ebenfalls Gegenstand der vorliegenden Anmeldung.

Es wurde auch gefunden, dass die Hydroxyalkylpyrazolylcarboxanilide der Formel (IV) sehr gute mikrobizide Eigenschaften besitzen und zur Bekämpfung unerwünschter Mikroorganismen sowohl im Pflanzenschutz als auch im Materialschutz eingesetzt werden können.

Die Hydroxyalkylpyrazolylcarboxanilide der Formel (IV) werden erhalten, indem man
f) Carbonsäure-Derivate der Formel (II) 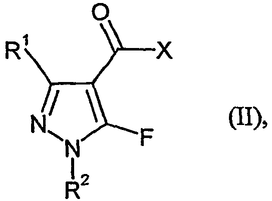 in welcher
   - R¹, R² und X: die oben angegebenen Bedeutungen haben,
   mit einem Hydroxyalkylanilinderivat der Formel (X) 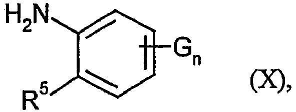 in welcher
   - R³, R⁵, G und n: die oben angegebenen Bedeutungen haben,
   gegebenenfalls in Gegenwart eines Säurebindemittels und gegebenenfalls in Gegenwart eines Verdünnungsmittels umsetzt.

Verwendet man 5-Fluor-1,3-dimethyl-1H-pyrazol-4-carbonyl-chlorid und 2-(2-Aminophenyl)-2-heptanol als Ausgangsstoffe, so kann der Verlauf des erfindungsgemäßen Verfahrens f) durch das folgende Formelschema veranschaulicht werden:

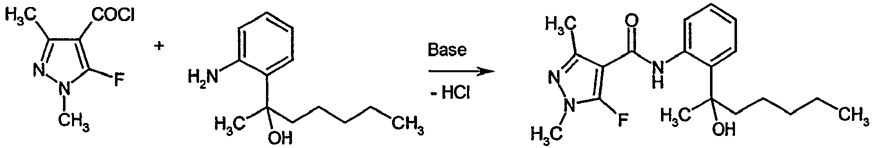

Die zur Durchführung des erfindungsgemäßen Verfahrens f) als Ausgangsstoffe benötigten Carbonsäure-Derivate der Formel (II) sind bereits weiter oben im Zusammenhang mit der Beschreibung des erfindungsgemäßen Verfahrens a) beschrieben worden.

Die zur Durchführung des erfindungsgemäßen Verfahrens f) als Ausgangsstoffe weiterhin benötigten Hydroxyalkylanilinderivate sind durch die Formel (X) allgemein definiert. In dieser Formel (X) haben R³, R⁵, G und n bevorzugt bzw. besonders bevorzugt diejenigen Bedeutungen, die bereits im Zusammenhang mit der Beschreibung der erfindungsgemäßen Verbindungen der Formeln (I) bzw. (IV) als bevorzugt bzw. als besonders bevorzugt für R³, R⁵, G und n angegeben wurden.

Die Hydroxyalkylanilinderivate der Formel (X) sind bekannt und/oder können nach bekannten Methoden erhalten werden (vgl. z.B. US 3,917,592 oder EP 0 824 099).

Verwendet man 5-Fluor-N-(2-iodphenyl)-1,3-dimethyl-1H-pyrazol-4-carboxamid und 1-Pentin oder alternativ 1-Hexen als Ausgangsstoffe, sowie jeweils einen Katalysator und eine Base, so kann der Verlauf des erfindungsgemäßen Verfahrens d) durch die beiden folgenden Formelschemata veranschaulicht werden:

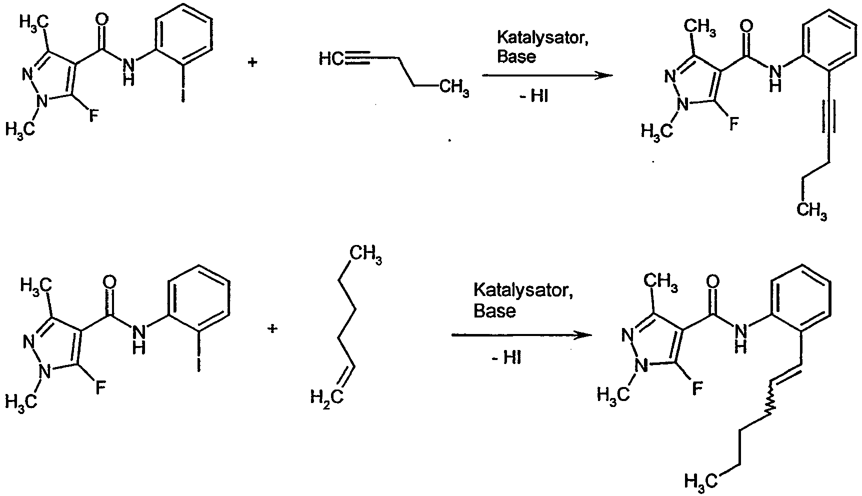

Die zur Durchführung des erfindungsgemäßen Verfahrens d) als Ausgangsstoffe benötigten Halogenpyrazolylcarboxanilide sind durch die Formel (V) allgemein definiert. In dieser Formel (V) haben R¹, R², G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für R¹, R², G und n angegeben wurden. Y steht bevorzugt für Brom oder Iod.

Die Halogenpyrazolylcarboxanilide der Formel (V) in denen (v) für Iod steht sind noch nicht bekannt, sie sind als neue Verbindungen ebenfalls Gegenstand der vorliegenden Anmeldung.

Sie werden erhalten, indem man
g) Carbonsäure-Derivate der Formel (II) 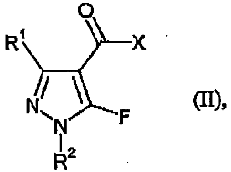 in welcher
   - R¹, R² und X: die oben angegebenen Bedeutungen haben,
   mit einem Halogenanilin der Formel (XI) 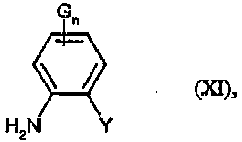 in welcher
   - G, n und Y: die oben angegebenen Bedeutungen haben,
   gegebenenfalls in Gegenwart eines Säurebindemittels und gegebenenfalls in Gegenwart eines Verdünnungsmittels umsetzt.

Verwendet man 5-Fluor-1,3-dimethyl-1H-pyrazol-4-carbonylchlorid und 2-Iodanilin als Ausgangsstoffe, so kann der Verlauf des erfindungsgemäßen Verfahrens g) durch das folgende Formelschema veranschaulicht werden:

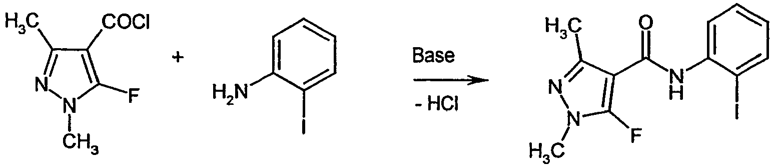

Die zur Durchführung des erfindungsgemäßen Verfahrens g) als Ausgangsstoffe benötigten Carbonsäure-Derivate der Formel (II) sind bereits weiter oben im Zusammenhang mit der Beschreibung des erfindungsgemäßen Verfahrens a) beschrieben worden.

Die zur Durchführung des erfindungsgemäßen Verfahrens g) als Ausgangsstoffe weiterhin benötigten Halogenaniline sind durch die Formel (XI) allgemein definiert. In dieser Formel (XI) haben G, n und Y bevorzugt bzw. besonders bevorzugt diejenigen Bedeutungen, die bereits im Zusammenhang mit der Beschreibung der erfindungsgemäßen Verbindungen der Formeln (I) bzw. (V) als bevorzugt bzw. besonders bevorzugt für G, n und Y angegeben wurden.

Die Halogenaniline der Formel (XI) sind bekannte Synthesechemikalien.

Die zur Durchführung des erfindungsgemäßen Verfahrens d) weiterhin als Ausgangsstoffe benötigten Alkine sind durch die Formel (VI) allgemein definiert.
- R⁶: steht bevorzugt für gegebenenfalls einfach bis vierfach, gleich oder verschieden durch Fluor, Chlor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- R⁶: steht besonders bevorzugt für jeweils gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Alkine der Formel (VI) sind bekannte Synthesechemikalien.

Die zur Durchführung des erfindungsgemäßen Verfahrens d) weiterhin alternativ als Ausgangsstoffe benötigten Alkene sind durch die Formel (VII) allgemein definiert.
- R⁷, R⁸ und R⁹: stehen unabhängig voneinander bevorzugt jeweils für Wasserstoff oder gegebenenfalls einfach bis vierfach, gleich oder verschieden durch Fluor, Chlor, Brom und/oder C₃-C₆-Cycloalkyl substituiertes Alkyl, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 12 nicht übersteigt.
- R⁷, R⁸ und R⁹: stehen unabhängig voneinander besonders bevorzugt jeweils für Wasserstoff oder gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl, wobei die Gesamtzahl der Kohlenstoffatome des offenkettigen Molekülteils die Zahl 12 nicht übersteigt.

Die Alkene der Formel (VII) sind bekannte Synthesechemikalien.

Verwendet man N-(2-Acetylphenyl)-5-fluor-1,3-dimethyl-1H-pyrazol-4-carboxamid und Butyl(triphenyl)-phosphonium-iodid als Ausgangsstoffe, so kann der Verlauf des erfindungsgemäßen Verfahrens e) durch das folgende Formelschema veranschaulicht werden:

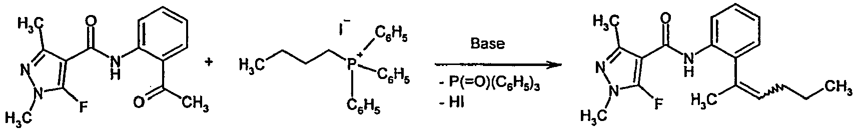

Die zur Durchführung des erfindungsgemäßen Verfahrens e) als Ausgangsstoffe benötigten Ketone sind durch die Formel (VIII) allgemein definiert. In dieser Formel (VIII) haben R¹, R², G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für R¹, R², G und n angegeben wurden.
- R¹⁰: steht bevorzugt für gegebenenfalls einfach bis vierfach, gleich oder verschieden durch Fluor, Chlor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- R¹⁰: steht besonders bevorzugt für jeweils gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Ketone der Formel (VIII) sind noch nicht bekannt. Sie sind als neue chemische Verbindungen ebenfalls Gegenstand der vorliegenden Anmeldung.

Sie werden erhalten, indem man
h) Carbonsäure-Derivate der Formel (II) 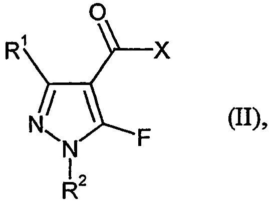 in welcher
   - R¹, R² und X: die oben angegebenen Bedeutungen haben,
   mit Ketoanilinen der Formel (XII) 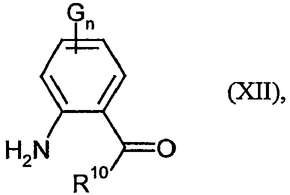 in welcher
   - R¹⁰, G und n: die oben angegebenen Bedeutungen haben,
   gegebenenfalls in Gegenwart eines Säurebindemittels und gegebenenfalls in Gegenwart eines Verdünnungsmittels umsetzt.

Verwendet man 5-Fluor-1,3-dimethyl-1H-pyrazol-4-carbonylchlorid und 1-(2-Aminophenyl)ethanon als Ausgangsstoffe, so kann der Verlauf des erfindungsgemäßen Verfahrens h) durch das folgende Formelschema veranschaulicht werden:

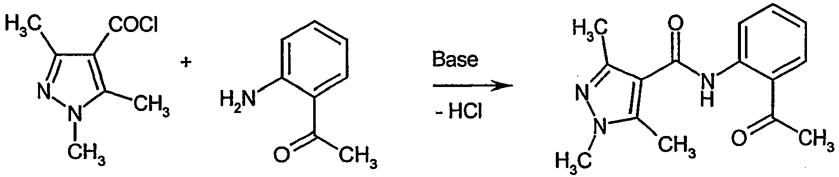

Die zur Dachführung des erfindungsgemäßen Verfahrens h) als Ausgangsstoffe benötigten Carbonsäure-Derivate der Formel (II) sind bereits weiter oben im Zusammenhang mit der Beschreibung des erfmdungsgemäßen Verfahrens a) beschrieben worden.

Die zur Durchführung des erfindungsgemäßen Verfahrens h) weiterhin als Ausgangsstoffe benötigten Ketoaniline sind durch die Formel (XII) allgemein definiert. In dieser Formel (XII) haben R¹⁰, G und n bevorzugt bzw. besonders bevorzugt diejenigen Bedeutungen, die bereits im Zusammenhang mit der Beschreibung der erfindungsgemäßen Verbindungen der Formeln (I) bzw. (VIII) als bevorzugt bzw. besonders bevorzugt für R¹⁰, G und n angegeben wurden.

Die Ketoaniline der Formel (XII) sind allgemein übliche Synthesechemikalien (vergleiche z. B. J. Am. Chem. Soc. 1978, 100(15), 4842-4857 oder US 4,032,573).

Die zur Durchführung des erfindungsgemäßen Verfahrens e) weiterhin als Ausgangsstoffe benötigten Phosphorverbindungen sind durch die Formel (IX) allgemein definiert.
- R¹¹: steht bevorzugt für gegebenenfalls einfach bis vierfach, gleich oder verschieden durch Chlor, Fluor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- R¹¹: steht besonders bevorzugt für jeweils gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.
- Px: steht bevorzugt für eine Gruppierung -P⁺(C₆H₅)₃ Cl-, -P⁺(C₆H₅)₃ Br⁻, -P⁺(C₆H₅)₃ I⁻, -P(==O)(OCH₃)₃ oder -P(=O)(OC₂H₅)₃.

Die Phosphorverbindungen der Formel (IX) sind bekannt und/oder können nach bekannten Verfahren hergestellt werden (vergleiche z. B. Justus Liebigs Ann. Chem. 1953, 580, 44-57 oder Pure Appl. Chem. 1964, 9, 307-335).

Als Verdünnungsmittel zur Durchführung der erfindungsgemäßen Verfahren a), f), g) und h) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren a), f), g) und h) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methyhnorpholin, N,N-Dimethylaminopyridin, Diazabicyclooctan (DABCO), Diazabicyclononen (DBN) oder Diazabicycloundecen (DBU).
Die Reaktionstemperaturen können bei der Durchführung der erfindungsgemäßen Verfahren a), f), g) und h) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens a) zur Herstellung der Verbindungen der Formel (I) setzt man pro mol des Carbonsäure-Derivates der Formel (II) im allgemeinen 0,2 bis 5 mol, vorzugsweise 0,5 bis 2 mol an Anilinderivat der Formel (III) ein.

Zur Durchführung des erfindungsgemäßen Verfahrens f) zur Herstellung der Verbindungen der Formel (IV) setzt man pro mol des Carbonsäure-Derivates der Formel (II) im allgemeinen 0,2 bis 5 mol, vorzugsweise 0,5 bis 2 mol an Hydroxyalkylanilinderivat der Formel (X) ein.

Zur Durchführung des erfindungsgemäßen Verfahrens g) zur Herstellung der Verbindungen der Formel (V) setzt man pro mol des Carbonsäure-Derivates der Formel (II) im allgemeinen 0,2 bis 5 mol, vorzugsweise 0,5 bis 2 mol an Halogenanilin der Formel (XI) ein.

Zur Durchführung des erfindungsgemäßen Verfahrens h) zur Herstellung der Verbindungen der Formel (VIII) setzt man pro mol des Carbonsäure-Derivates der Formel (II) im allgemeinen 0,2 bis 5 mol, vorzugsweise 0,5 bis 2 mol an Ketoanilin der Formel (XII) ein.

Als Verdünnungsmittel zur Durchführung des erfindungsgemäßen Verfahrens b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether.

Das erfindungsgemäße Verfahren b) wird gegebenenfalls in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Hydrierung im erfindungsgemäßen Verfahren b) kann statt in Gegenwart von Wasserstoff in Kombination mit einem Katalysator auch in Anwesenheit von Triethylsilan durchgeführt werden.

Die Reaktionstemperaturen können bei der Durchführung des erfindungsgemäßen Verfahrens b) in einem größeren Bereich variiert werden. Im allgemeinen arbeitet man bei Temperaturen von 0°C bis 150°C, vorzugsweise bei Temperaturen von 0°C bis 80°C.

Als Verdünnungsmittel zur Durchführung des erfindungsgemäßen Verfahrens c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren c) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des erfindungsgemäßen Verfahrens c) in einem größeren Bereich variiert werden. Im allgemeinen arbeitet man bei Temperaturen von 0°C bis 150°C, vorzugsweise bei Temperaturen von 0°C bis 80°C.

Die erfindungsgemäßen Verfahren c) und b) können auch in einer Tandemreaktion ("Eintopf-Reaktion") durchgeführt werden. Dazu wird eine Verbindung der Formel (IV) gegebenenfalls in Gegenwart eines Verdünnungsmittels (geeignete Lösungsmittel wie für Verfahren c)), gegebenenfalls in Gegenwart einer Säure (geeignete Säuren wie für Verfahren c)) und in Anwesenheit von Triethylsilan umgesetzt.

Als Verdünnungsmittel zur Durchführung des erfindungsgemäßen Verfahrens d) kommen alle inerten organischen Lösungsmittel in Betracht. Hierzu gehören vorzugsweise Nitrile, wie Acetonitril, Propionitril, n- oder i-Butyronitril oder Benzonitril oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren d) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren d) wird in Gegenwart eines oder mehrerer Katalysatoren durchgeführt.

Dazu eignen sich besonders Palladiumsalze oder -komplexe. Hierzu kommen vorzugsweise Palladiumchlorid, Palladiumacetat, Tetrakis-(triphenylphosphin)-Palladium oder Bis-(triphenylphosphin)-Palladiumdichlorid infrage. Es kann auch ein Palladiumkomplex in der Reaktionsmischung erzeugt werden, wenn man ein Palladiumsalz und ein Komplexligand getrennt zur Reaktion zugibt.

Als Liganden kommen vorzugsweise Organophosphorverbindungen infrage. Beispielhaft seien genannt: Triphenylphosphin, tri-o-Tolylphosphin, 2,2'-Bis(diphenylphosphino)-1,1'-binaphthyl, Dicyclohexylphosphinbiphenyl, 1,4-Bis(diphenylphosphino)butan, Bisdiphenylphosphinoferrocen, Di(tert.-butylphosphino)biphenyl, Di-(cyclohexylphosphino)biphenyl, 2-Dicydohexylphosphino-2'-N,N-dimethylaminobiphenyl, Tricyclohexylphosphin, Tri-tert.-butylphosphin. Es kann aber auch auf Liganden verzichtet werden.

Das erfindungsgemäße Verfahren d) wird ferner gegebenenfalls in Gegenwart eines weiteren Metallsalzes, wie Kupfersalzen, beispielsweise Kupfer-(I)-iodid durchgeführt.

Die Reaktionstemperaturen können bei der Durchführung des erfindungsgemäßen Verfahrens d) in einem größeren Bereich variiert werden. Im allgemeinen arbeitet man bei Temperaturen von 20°C bis 180°C, vorzugsweise bei Temperaturen von 50°C bis 150°C.

Zur Durchführung des erfindungsgemäßen Verfahrens d) zur Herstellung der Verbindungen der Formel (I) setzt man pro mol des Halogenpyrazolylcarboxanilids der Formel (V) im allgemeinen 1 bis 5 mol, vorzugsweise 1 bis 2 mol an Alkin der Formel (VI) oder Alken der Formel (VII) ein.

Als Verdünnungsmittel zur Durchführung des erfindungsgemäßen Verfahrens e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether.

Das erfindungsgemäße Verfahren e) wird gegebenenfalls in Gegenwart eines geeigneten Säureakzeptors durchgeführt. Als solche kommen alle üblichen starken Basen infrage. Hierzu gehören vorzugsweise Erdalkalimetall- oder Alkalimetallhydride, -hydroxide, -amide, -alkoholate oder Alkalimetall-Kohlenwasserstoffverbindungen, wie beispielsweise Natriumhydrid, Natriumhydroxid, Kaliumhydroxid, Natriumamid, Lithiumdiisopropylamid, Natrium-methylat, Natrium-ethylat, Kalium-tert.-butylat, Methyllithium, Phenyllithium oder Butyllithium.

Die Reaktionstemperaturen können bei der Durchführung des erfindungsgemäßen Verfahrens e) in einem größeren Bereich variiert werden. Im allgemeinen arbeitet man bei Temperaturen von -80°C bis 150°C, vorzugsweise bei Temperaturen von -30°C bis 80°C.

Zur Durchführung des erfindungsgemäßen Verfahrens e) zur Herstellung der Verbindungen der Formel (I) setzt man pro mol des Ketons der Formel (VIII) im allgemeinen 1 bis 5 mol, vorzugsweise 1 bis 2 mol an Phosphorverbindung der Formel (IX) ein.

Alle erfindungsgemäßen Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.
Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Pyrenophora-Arten, von Krankheiten im Wein-, Obst- und Gemüseanbau, wie beispielsweise gegen Altemaria - oder Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Altemaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.
Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Edifenphos, Epoxiconazol, Etaconazol, Ethirimol, Etridiazol,
   Famoxadon, Fenapanil, Fenarimol, Fenbuconazol, Fenfuram, Fenhexamid, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin, Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Iprovalicarb, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
   Quinconazol, Quintozen (PCNB), Quinoxyfen,
   Schwefel und Schwefel-Zubereitungen, Spiroxamine,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol,
   Validamycin A, Vinclozolin, Viniconazol,
   Zarilamid, Zineb, Ziram sowie
   Dagger G, OK-8705, OK-8801,
   α-(1,1-Dimethylethyl)-β-(2-phenoxyethyl)-1H-1,2,4-triazol-1-ethanol,
   α-(2,4-Dichlorphenyl)-β-fluor-β-propyl-1H-1,2,4-triazol-1-ethanol,
   α-(2,4-Dichlorphenyl)-β-methoxy-α-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-α-(Methoxyimino)-N-methyl-2-phenoxy-phenylacetamid,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
   2-Aminobutan, 2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion, 3,5-Dichlor-N-[cyan[(1-methyl-2-propynyl)-oxy]-methyl]-benzamid,
   3-(1,1-Dimethylpropyl)-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
   4-[(3,4-Dimethoxyphenyl)-3-(4-fluorphenyl)-acryloyl]-morpholin
**Bakterizide:**
   Bronopol, Dichloroplien, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene, Imidacloprid, Indoxacarb, Isazofos, Isofenphos, Isoxathion, Ivermectin, Kernpolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos, Ribavirin,
   Salithion, Sebufos, Silafluofen, Spinosad, Spirodiclofen, Sulfotep, Sulprofos, Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidoihion, Vaniliprole, Verticillium lecanii
   YI 5302, Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol 2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid 3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3-(2H)-pyridazinon Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Herstellungsbeispiele

### Beispiel 1

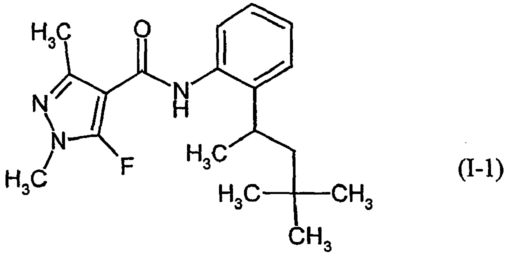

### Verfahren (a):

Eine Mischung aus 382,6 mg (2 mmol) 2-(1,3,3-Trimethylbutyl)anilin, 353,2 mg (2 mmol) 5-Fluor-1,3-dimethyl-1H-pyrazol-4-carbonsäurechlorid und 276,4 mg (2 mmol) Kaliumcarbonat in 25 ml Acetonitril wird über Nacht bei Raumtemperatur gerührt. Es wird 30 ml gesättigte Ammoniumchloridlösung zugegeben und mit 30 ml Essigsäureethylester extrahiert. Die vereinigten organischen Phasen werden über Magnesiumsulfat getrocknet und bei vermindertem Druck eingeengt. Der Rückstand wird mit Cylohexan/Essigsäureethylester (beginnend mit reinem Cyclohexan und zuletzt mit 80 % Essigsäureethylester) an Kieselgel chromatographiert.

Man erhält 280 mg (42 % der Theorie) 5-Fluor-1,3-dimethyl-N-[2-(1,3,3-trimethylbutyl)phenyl]-1H-pyrazol-4-carboxamid vom Schmelzpunkt 78 - 80°C.

### Beispiel 2

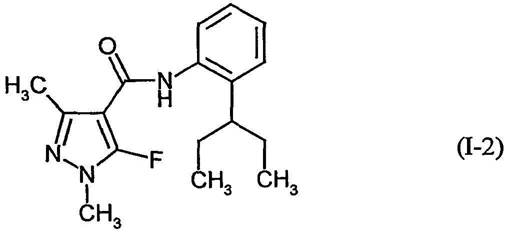

### Verfahren (b)

540 mg (1,792 mmol) N-[2-(1-Ethenyl-propyl)-phenyl]-5-fluor-1,3-dimethyl-1H-pyrazol-4-carboxamid (1-24) werden in 50 mL Methanol in Anwesenheit von 500 mg 5% Pd/C bei 80°C für 5 Stunden unter Wasserstoff-Druck von 100 bar hydriert. Der Katalysator wird abfiltriert, der Filterrückstand mit 2 x 50 mL Methanol gewaschen und die Mutterlauge unter vermindertem Druck eingeengt. Der Rückstand wird mit Cyclohexan/Essigsäureethylester 4:1 an Kieselgel chromatographiert.

Man erhält 150 mg (30% der Theorie) N-[2-(1-Ethyl-propyl)-phenyl]-5-fluor-1,3-dimethyl-1H-pyrazol-4-carboxamid als fahlgelbe Kristalle.

HPLC: logP = 3,01

### Beispiel 3

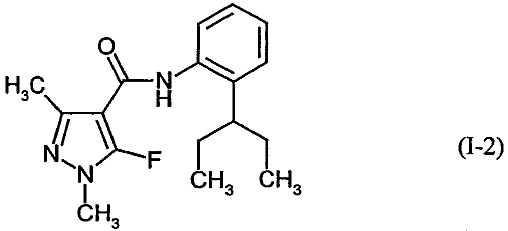

### Verfahren (c) und (b) als Tandemreaktion

450 mg (1,41 mmol) N-[2-(1-Ethyl-1-hydroxy-propyl)-phenyl]-5-fluor-1,3-dimethyl-1H-pyrazol-4-carboxamid (IV-1) werden in 10 mL Dichlormethan unter Zugabe von 1,6g (14,1 mmol) Trifluoressigsäure und 0,16 g (1,41 mmol) Triethylsilan bei Raumtemperatur für 1 Stunde gerührt. Die Reaktionslösung wird mit gesättigter Natriumhydrogencarbonat-Lösung auf pH 7 gebracht und die organische Phase abgetrennt, über Magnesiumsulfat getrocknet und unter vermindertem Druck eingeengt. Der Rückstand wird mit Cyclohexan/Essigsäureethylester 4:1 an Kieselgel chromatographiert.

Man erhält 120 mg (20% der Theorie) N-[2-(1-Ethyl-propyl)-phenyl]-5-fluor-1,3-dimethyl-1H-pyrazol-4-carboxamid als fahlgelbe Kristalle.

HPLC: logP = 3,01

### Beispiel 4

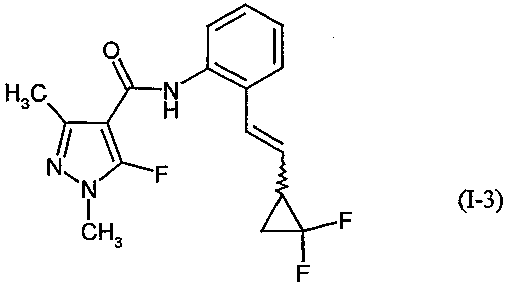

### Verfahren (d)

0,718 g (2 mmol) des Iodanilids 5-Fluor-N-(2-iodphenyl)-1,3-dimethyl-1H-pyrazol-4-carboxamid, 0,25 g (2,5 mmol) 1,1-Difluor-2-vinylcyclopropan und 0,33 ml (2,4 mmol) Triethylamin werden in 4 ml Dimethylformamid gelöst, anschließend wird 5 Minuten lang Argon durch die Reaktionslösung geleitet. Nun werden noch 20 mg (0,09 mmol) Palladiumacetat zugegeben und für 16 Stunden bei 100°C im luftdicht verschlossenen Gefäß gerührt. Nach Ende der Reaktion wird die Mischung auf eine Silicakartusche gegeben und mit Essigsäureethylester eluiert. Das Eluat wird mit Aktivkohle versetzt, filtriert und bei vermindertem Druck eingeengt. Der Rückstand wird mit Cyclohexan/Essigester (3:1 bis 1:1) an Kieselgel chromatographiert.

Man erhält 154 mg (23 % der Theorie) N-{2-[2-(2,2-Difluorcyclopropyl)ethenyl]phenyl}-5-fluor-1,3-dimethyl-1H-pyrazol-4-carboxamid.

HPLC: logP = 2,59

### Beispiel 5

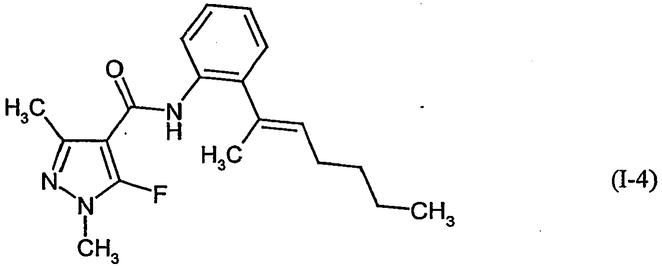

### Verfahren (e):

227 mg (0,55 mmol) n-Butyl-triphenylphosphoniumbromid werden in 2 ml Tetrahydrofuran suspendiert, bei -30°C mit 0,34 ml (0,55 mmol) einer n-BuLi-Lösung in Hexan versetzt und 20 min bei dieser Temperatur gerührt. Nun wird eine Suspension von 138 mg (0,5 mmol) N-(2-Acetylphenyl)-5-fluor-1,3-dimethyl-1H-pyrazol-4-carboxamid in 2 ml Tetrahydrofuran zugegeben und ohne weitere Kühlung für 16 Stunden gerührt. Es werden 3 ml Wasser zugegeben und 3 Mal mit jeweils 20 ml Essigsäureethylester extrahiert. Die vereinigten organischen Phasen werden über Magnesiumsulfat getrocknet und bei vermindertem Druck eingeengt. Der Rückstand wird mit Cyclohexan/Essigester (5:1 bis 1:1) an Kieselgel chromatographiert.

Man erhält 17mg (10 % der Theorie) 5-Fluor-1,3-dimethyl-N-[2-(1-methyl-1-hexenyl)phenyl]-1H-pyrazol-4-carboxamid.

HPLC: logP = 4,36

Analog den Beispielen 1 bis 5, sowie entsprechend den Angaben in den allgemeinen Verfahrensbeschreibungen, werden die in der nachstehenden Tabelle 1 genannten Verbindungen der Formel (I) erhalten.

| Bsp.Nr. | Verbindung | logP | Fp. (°C) |
|---|---|---|---|
| I-5 | 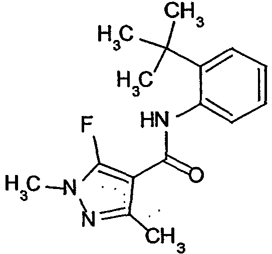 | | |
| I-6 | 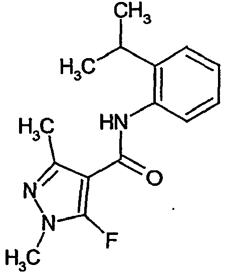 | | 114 |
| I-7 | 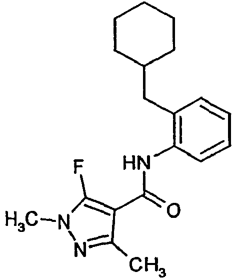 | | 92-94 |
| I-8 | 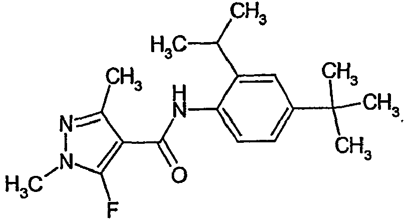 | | |
| I-9 | 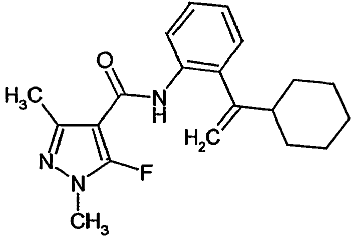 | 4,36 | 132-134 |
| I-10 | 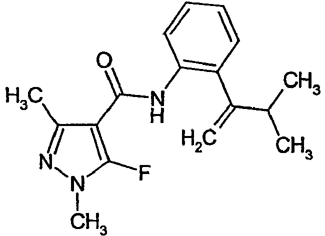 | 3,37 | 109-111 |
| I-11 | 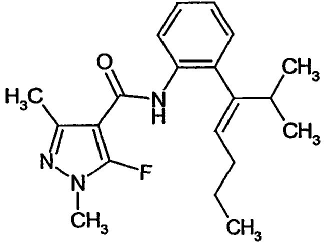 | | |
| I-12 | 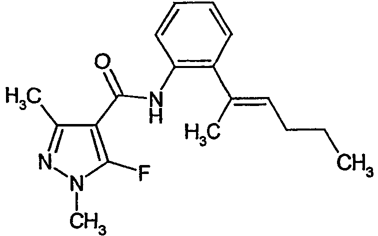 | | 97 |
| I-13 | 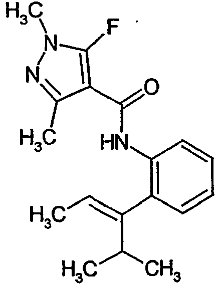 | | 143 |
| I-14 | 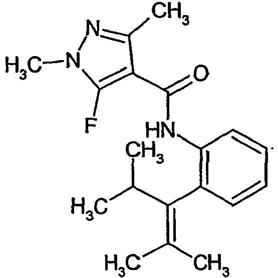 | | 151 |
| I-15 | 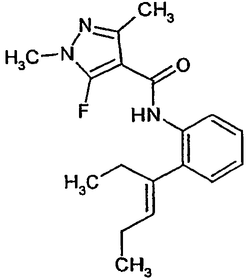 | | |
| I-16 | 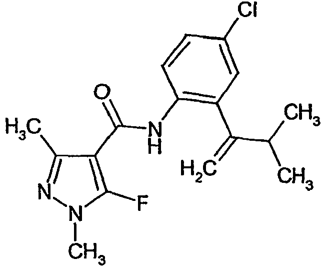 | 4,11 | 155-157 |
| I-17 | 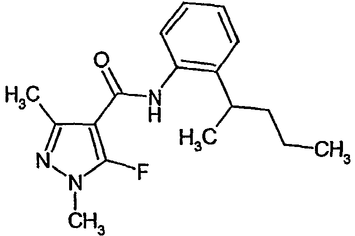 | 3,01 | 84-87 |
| I-18 | 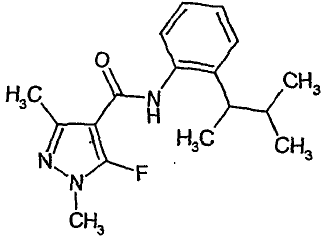 | 2,92 | 85 |
| I-19 | 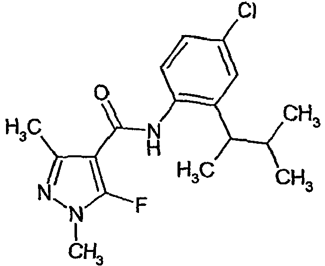 | 3,43 | 123-124 |
| I-20 | 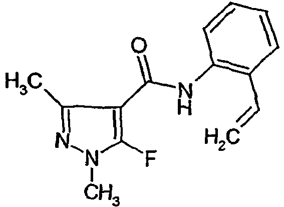 | 1,92 | |
| I-21 | 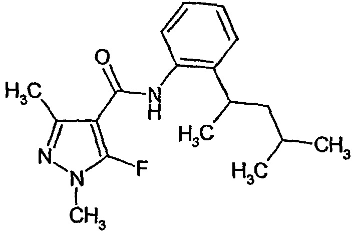 | 3,26 | |
| I-22 | 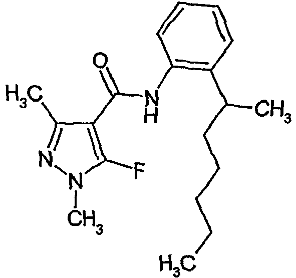 | 3,83 | |
| I-23 | 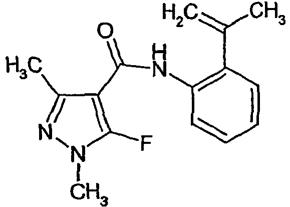 | 2,57 | |
| I-24 | 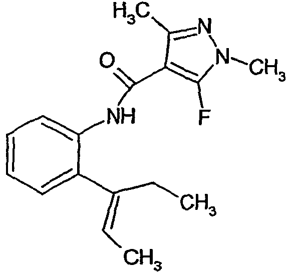 | 3,45 | |
| I-25 | 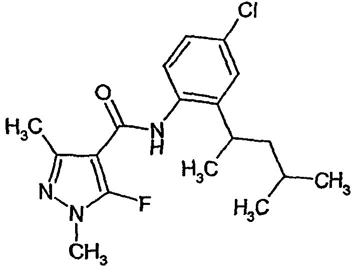 | 3,82 | 111-13 |
| I-26 | 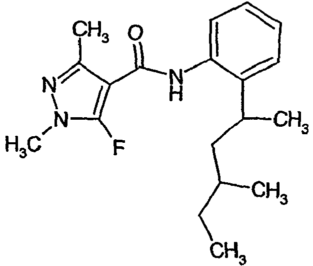 | 3,67 | |
| I-27 | 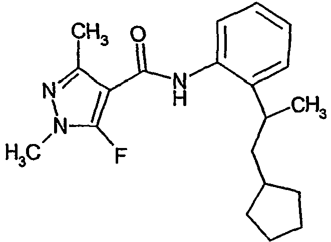 | 3,82 | |
| I-28 | 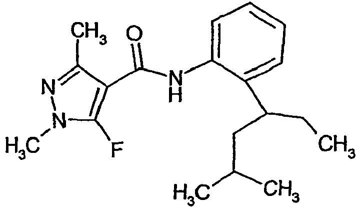 | 3,73 | |
| I-29 | 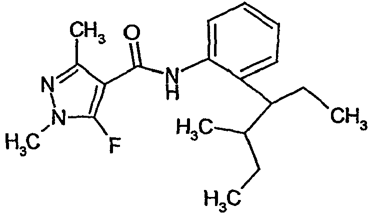 | 3,74 | |
| I-30 | 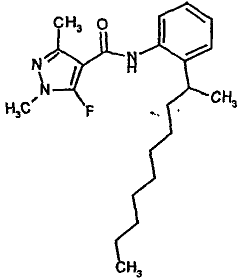 | 5,29 | |
| I-31 | 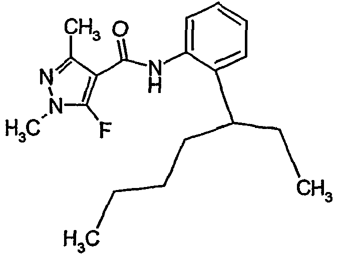 | 3,85 | |
| I-32 | 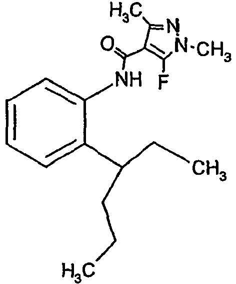 | 3,42 | |
| I-33 | 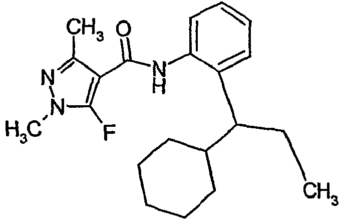 | 4,34 | |
| I-34 | 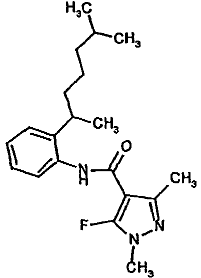 | 4,22 | |
| I-35 | 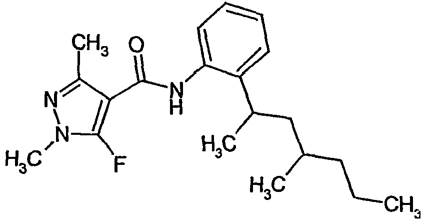 | 4,11 | |
| I-36 | 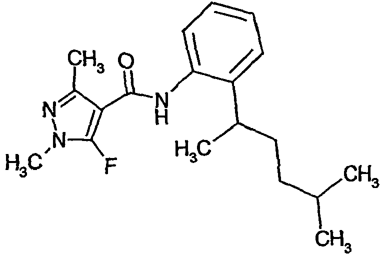 | 3,76 | |
| I-37 | 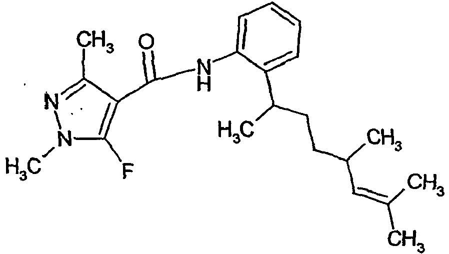 | 4,49 | |
| I-38 | 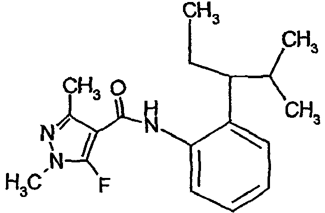 | 3,37 | |
| I-39 | 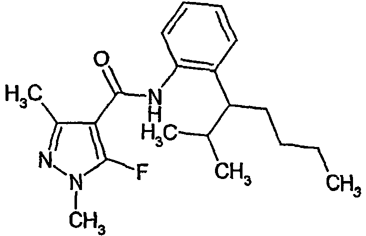 | 4,24 | |
| I-40 | 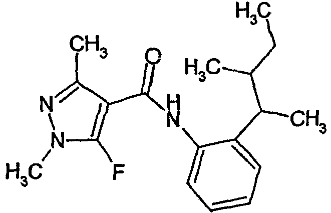 | 3,28 | |
| I-41 | 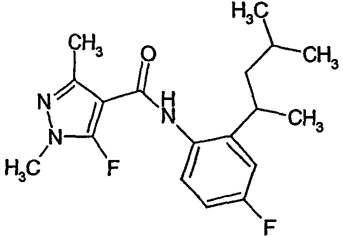 | 3,32 | |
| I-42 | 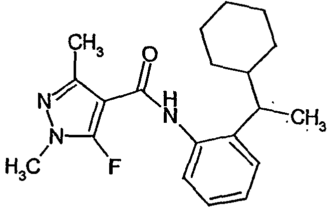 | 3,82 | |
| I-43 | 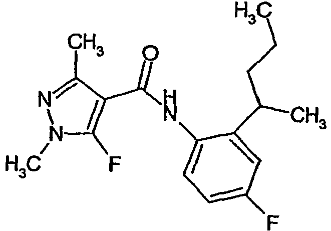 | 3,05 | |
| I-44 | 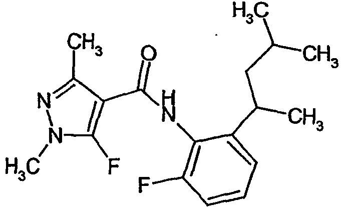 | 3,16 | |
| I-45 | 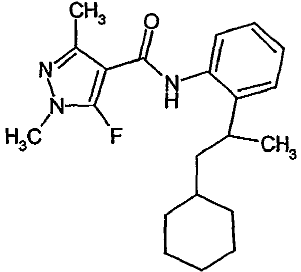 | 4,2 | |
| I-46 | 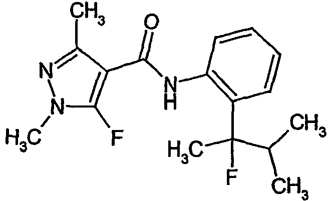 | 3,09 | |
| I-47 | 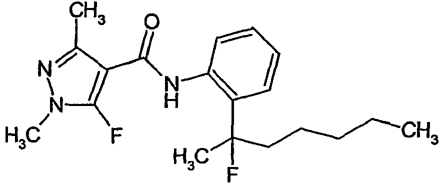 | 4,01 | |
| I-48 | 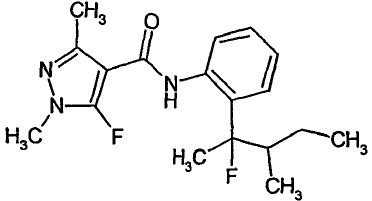 | 3,44 | |
| I-49 | 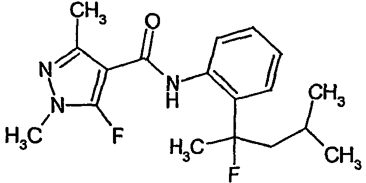 | 3,47 | |
| I-50 | 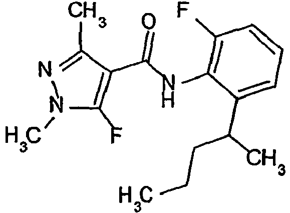 | 2,86 | |
| I-51 | 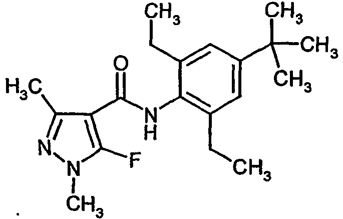 | | |
| I-52 | 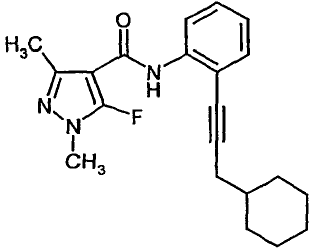 | 5,08 | |
| I-53 | 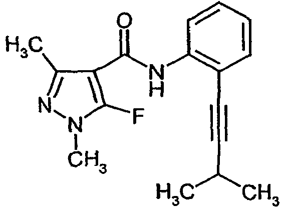 | 3,59 | |
| I-54 | 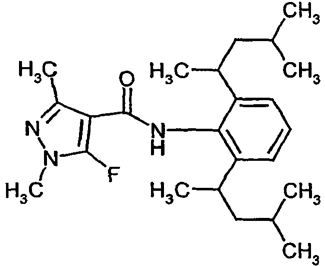 | 4,90 | |

### Herstellung der Zwischenprodukte

### Beispiel (IV-1)

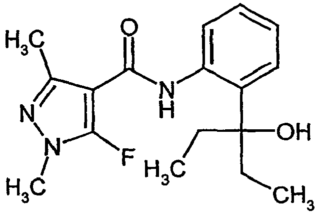

### Verfahren (f):

358,5 mg (2 mmol) 3-(2-Aminophenyl)-3-pentanol und 353,2 mg (2 mmol) 5-Fluor-1,3-dimethyl-1H-pyrazol-4-earbonsäurechlorid werden mit 276,4 mg (2 mmol) Kaliumcarbonat in 15 ml Acetonitril bei Raumtemperatur für 16 Stunden gerührt. Nach Zugabe von 30 ml gesättigter Ammoniumchloridlösung wird mit 30 ml Essigsäureethylester extrahiert. Die vereinigten organischen Phasen werden mit 30 ml konzentrierter Natriumchloridlösung gewaschen, über Magnesiumsulfat getrocknet und bei vermindertem Druck eingeengt. Der Rückstand wird mit Cylohexan/Essigsäureethylester (beginnend mit reinem Cyclohexan und zuletzt mit 80 % Essigsäureethylester) an Kieselgel chromatographiert.

Man erhält 350 mg (45 % der Theorie) N-[2-(1-Ethyl-1-hydroxypropyl)phenyl]-5-fluor-1,3-dimethyl-1H-pyrazol-4-carboxamid als farblose Kristalle.

HPLC:logP=2,47

Analog Beispiel (IV-1) sowie entsprechend den Angaben in den allgemeinen Verfahrensbeschreibungen, werden die in der nachstehenden Tabelle 2 genannten Verbindungen der Formel (IV) erhalten.

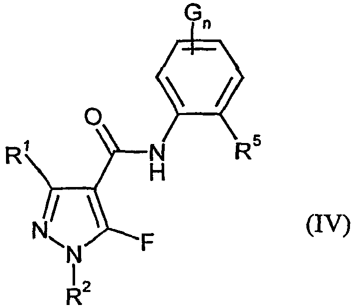

| Bsp.Nr. | Verbindung | logP | Fp. (°C) |
|---|---|---|---|
| IV-2 | 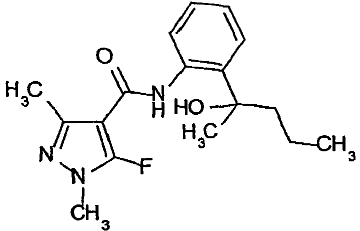 | 2,4 | 158 |
| IV-3 | 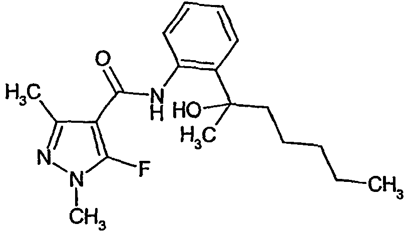 | 3,13 | |
| IV-4 | 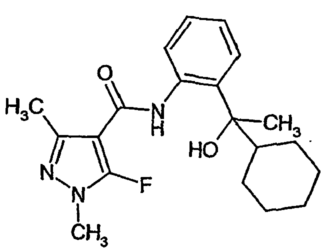 | 3,1 | 227 |
| IV-5 | 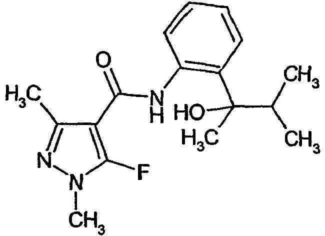 | 2,41 | 162-164 |
| IV-6 | 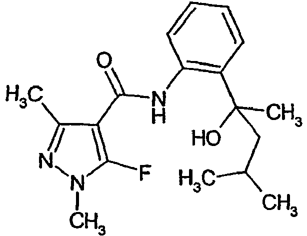 | 2,68 | 150-152 |
| IV-7 | 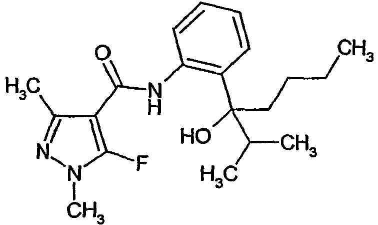 | | 173 |
| IV-8 | 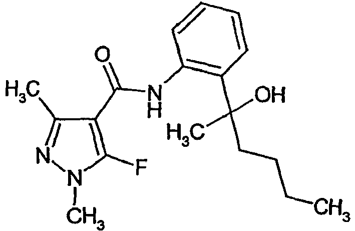 | | 161 |
| IV-9 | 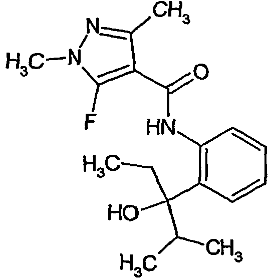 | | 165 |
| IV-10 | 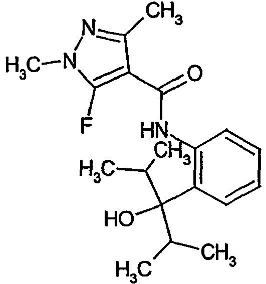 | | 181 |
| IV-11 | 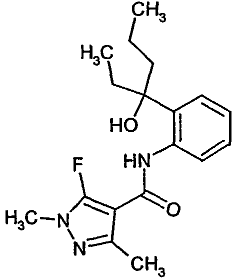 | | 167 |
| IV-12 | 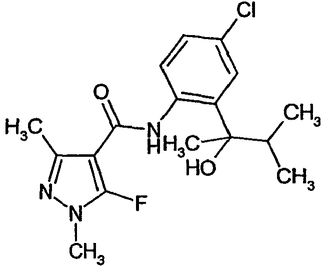 | 2,99 | |
| IV-13 | 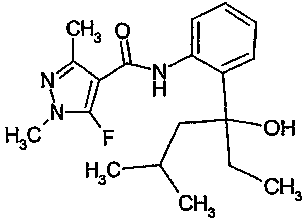 | 3,11 | |

### Beispiel (V-1)

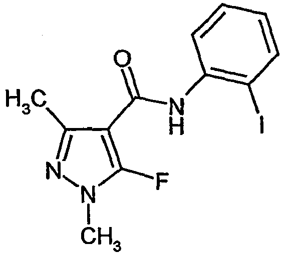

### Verfahren (g):

Eine Mischung von 5,86 g (42 mmol) Kaliumcarbonat und 6,2 g (28,3 mmol) 2-Iodanilin in 100 ml Acetonitril wird 10 min bei Raumtemperatur gerührt. Hierzu wird eine Lösung aus 5 g (28,3 mmol) 5-Fluor-1,3-dimethyl-1H-pyrazol-4-carbonyl chlorid in 10 ml Acetonitril langsam zugegeben und für 16 Stunden unter Rückfluss gerührt. Die Reaktionsmischung wird unter vermindertem Druck eingeengt und mit 200 ml Essigsäureethylester und 100 ml Wasser versetzt. Die organische Phase wird abgetrennt und die wässrige Phase 3 Mal mit 200 ml Essigsäureethylester extrahiert. Die vereinigten organischen Phasen werden über Magnesiumsulfat getrocknet und bei vermindertem Druck eingeengt. Der Rückstand wird in 50 ml Ether verrührt.

Man erhält 4,78 g (47 % der Theorie) 5-Fluor-N-(2-iodphenyl)-1,3-dimethyl-1H-pyrazol-4-carboxamid.

HPLC: logP = 2,44

### Beispiel (VIII-1)

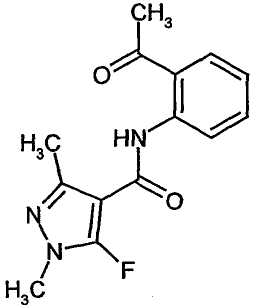

### Verfahren (h):

Zu einer Mischung von 10,4 g (76 mmol) Kaliumcarbonat und 5,1 g (38 mmol) 2-Aminoacetophenon in 40 ml Acetonitril wird eine Lösung aus 8 g (45 mmol) 5-Fluor-1,3-dimethyl-1H-pyrazol-4-carbonyl chlorid in 30 ml Acetonitril langsam zugegeben und für 16 Stunden bei 80°C gerührt. Die Reaktionsmischung wird unter vermindertem Druck eingeengt und mit 200 ml Essigsäureethylester und 70 ml Wasser versetzt. Die organische Phase wird abgetrennt und die wässrige Phase 3 mal mit 200 ml Essigsäureethylester extrahiert. Die vereinigten organischen Phasen werden über Magnesiumsulfat getrocknet und bei vermindertem Druck eingeengt. Der Rückstand wird über Kieselgelkartusche mit Dichlormethan filtriert, das Filtrat bei vermindertem Druck eingeengt und das Rohprodukt mit Diisopropylether verrühren.

Man erhält 2,5 g (24 % der Theorie) N-(2-Acetylphenyl)-5-fluor-1,3-dimethyl-1H-pyrazol-4-carboxamid.

HPLC: logP = 2,15

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Pyrenophora teres-Test (Gerste) / protektiv

| | | |
|---|---|---|
| Lösungsmittel: | 25 | Gewichtsteile N,N-Dimethylacetamid |
| Emulgator: | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die Verbindungen 16 und 17 der Herstellungsbeispiele bei einer Aufwandmenge von 250 g/ha einen Wirkungsgrad von 95 % oder mehr.

**Tabelle A**

| Pyrenophora teres-Test (Gerste) / protektiv | | |
|---|---|---|
| Wirkstoff Erfindungsgemäß | Aufwandmenge an Wirkstoff in g/ha | Wirkungsgrad in % |
| 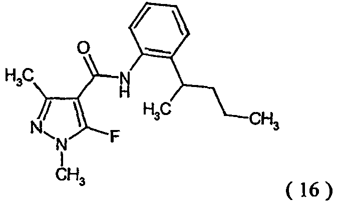 | 250 | 100 |
| 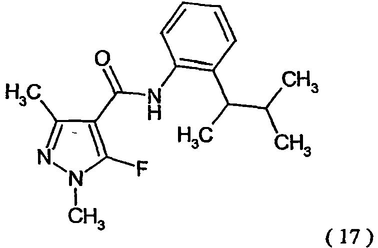 | 250 | 100 |

### Beispiel B

### Podosphaera-Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die Verbindungen 9,16, 17 und 18 der Herstellungsbeispiele bei einer Aufwandmenge von 100 g/ha einen Wirkungsgrad von 90 % oder mehr.

**Tabelle B**

| Podosphaera-Test (Apfel) / protektiv | | |
|---|---|---|
| Wirkstoff Erfindungsgemäß | Aufwandmenge an Wirkstoff in g/ha | Wirkungsgrad in % |
| 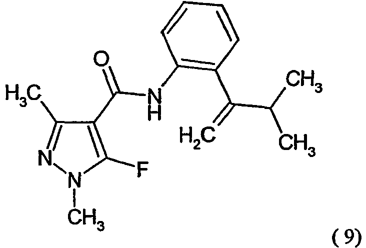 | 100 | 90 |
| 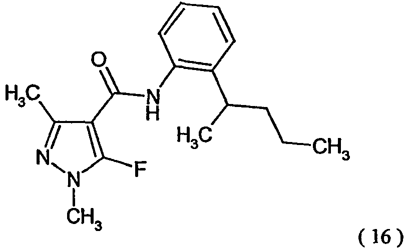 | 100 | 100 |
| 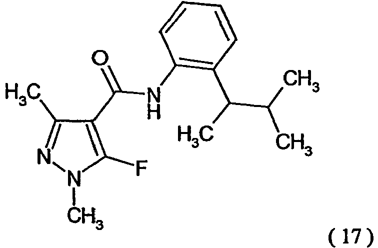 | 100 | 100 |
| 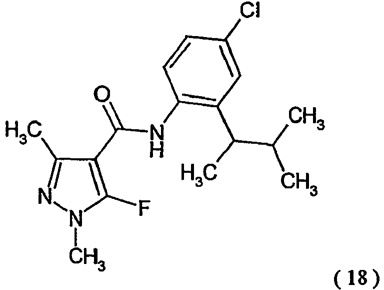 | 100 | 100 |

### Beispiel C

### Alternaria-Test (Tomate) / protektiv

| | | |
|---|---|---|
| Lösungsmittel: | 49 | Gewichtsteile N,N-Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Altemaria solani inokuliert und stehen dann 24h bei 100 % rel. Feuchte und 20°C. Anschließend stehen die Pflanzen bei 96 % rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die Verbindungen 16, 17 und 20 der Herstellungsbeispiele bei einer Aufwandmenge von 750 g/ha einen Wirkungsgrad von 90 % oder mehr.

**Tabelle C**

| Alternaria-Test (Tomate) / protektiv | | |
|---|---|---|
| Wirkstoff | Aufwandmenge an Wirkstoff in g/ha | Wirkungsgrad in % |
| 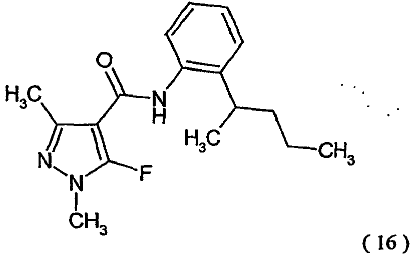 | 750 | 95 |
| 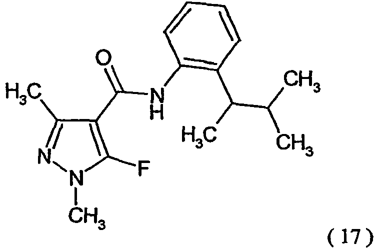 | 750 | 95 |
| 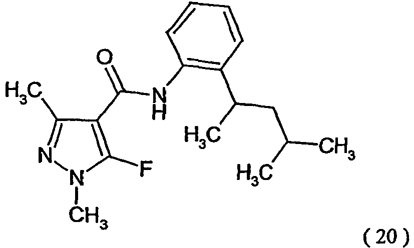 | 750 | 100 |

## Patentansprüche

1. Pyrazolylcarboxanilide der formel (I) 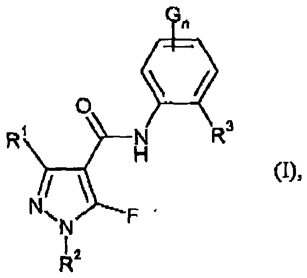 in welcher
R¹ für Wasserstoff, Cyano, Halogen, Nitro, C₁-C₄-Alkyl, C₁-C₄-Halogenalkyl mit 1 bis 5 Halogenatomen, C₃-C₆-Cycloalkyl, C₁-C₄-Alkoxy, C₁-C₄-Halogenalkoxy mit 1 bis 5 Halogenatomen, C₁-C₄-Alkyl-thio, C₁-C₄-Halogenalkylthio mit 1 bis 5 Halogenatomen oder Aminocarbonyl-C₁-C₄-alkyl steht,
R² für Wasserstoff, C₁-C₄-Alkyl, C₁-C₆-Halogenalkyl mit 1 bis 5 Halogenatomen, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Halogenatomen, C₁-C₄-Alkoxy-C₁-C₄-alkyl oder C₁-C₄-Halogenalkoxy-C₁-C₄-alkyl mit 1 bis 5 Halogenatomen steht,
G für Halogen oder C₁-C₄-Alkyl steht,
G außerdem für C₅-C₆-Alkyl steht,
R³ für unsubstituiertes C₅-C₂₀-Alkyl oder für einfach oder mehrfach, gleich oder verschieden durch Halogen und/oder C₃-C₆-Cycloalkyl substituiertes C₁-C₂₀-Alkyl oder für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann und
n für 0, 1 oder 2 steht.

2. Pyrazolylcarboxanilide der Formel (I) gemäß Anspruch 1, in welcher
G für Halogen oder C₁-C₄-Alkyl steht,
und R¹, R², R³ und n die in Anspruch 1 angegebenen Bedeutungen haben.

3. Pyrazolylcarboxanilide der Formel (I) gemäß Anspruch 1, in welcher
R¹ für Wasserstoff, Cyano, Halogen, Nitro, C₁-C₄-Alkyl, C₁-C₄-Halogenalkyl mit 1 bis 5 Fluor-, Chlor- und/oder Bromatomen, C₃-C₆-Cycloalkyl, C₁-C₄-Alkoxy, C₁-C₄-Halogenalkoxy mit 1 bis 5 Fluor-, Chlor- und/oder Bromatomen, C₁-C₄-Alkylthio, C₁-C₄-Halogenalkyl-thio mit 1 bis 5 Fluor-, Chlor- und/oder Bromatomen oder Aminocarbonyl-C₁-C₄-alkyl steht,
R² für Wasserstoff, C₁-C₄-Alkyl, C₁-C₆-Halogenalkyl mit 1 bis 5 Fluor-, Chlor- und/oder Bromatomen, C₂-C₆-Alkenyl, C₃-C₆-Cycloalkyl, C₁-C₄-Alkylthio-C₁-C₄-alkyl, C₁-C₄-Halogenalkylthio-C₁-C₄-alkyl mit 1 bis 5 Fluor-, Chlor- und/oder Bromatomen, C₁-C₄-Alkoxy-C₁-C₄-alkyl oder C₁-C₄-Halogenalkoxy-C₁-C₄-alkyl mit 1 bis 5 Fluor-, Chlor- und/oder Bromatomen steht,
G für Halogen oder C₁-C₄-Alky] steht,
G außerdem für C₅-C₆-Alkyl steht,
R³ für unsubstituiertes C₆-C₁₂-Alkyl oder für einfach bis vierfach, gleich oder verschieden durch Fluor, Chlor, Brom und/oder C₃-C₆-Cycloalkyl substituiertes C₂-C₁₂-Alkyl oder für jeweils gegebenenfalls einfach bis vierfach, gleich oder verschieden durch Fluor, Chlor, Brom und/oder C₃-C₆-Cycloalkyl substituiertes C₂-C₁₂-Alkenyl oder C₂-C₁₂-Alkinyl steht, wobei der Cycloalkylteil seinerseits gegebenenfalls einfach bis vierfach, gleich oder verschieden durch Halogen und/oder C₁-C₄-Alkyl substituiert sein kann,
n für 0, 1 oder 2 steht.

4. Pyrazolylcarboxanilide der Formel (I) gemäß Anspruch 3, in welcher
G für Halogen oder C₁-C₄-Alkyl steht,
und R¹, R², R³ und n die in Anspruch 3 angegebenen Bedeutungen haben.

5. Pyrazolylcarboxanilide der Formel (I) gemäß Anspruch 1, in welcher
R¹ für Wasserstoff, Methyl, Ethyl, n- oder i-Propyl, n-, i-, s- oder t-Butyl, Trifluormethyl oder Trifluorethyl steht,
R² für Wasserstoff, Methyl, Ethyl, n- oder i-Propyl, n-, i-, s- oder t-Butyl, Trifluormethyl oder Trifluorethyl steht,
G für Fluor, Chlor oder Methyl steht,
G außerdem für Ethyl oder t-Butyl steht,
G außerdem für 2,4-Dimethylbutyl steht,
R³ für jeweils gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl, Octyl, Nonyl, Decyl, Ethenyl, Propenyl, Butenyl, Pentenyl, Hexenyl, Heptenyl, Octenyl, Nonenyl, Decenyl, Ethinyl, Propinyl, Butinyl, Pentinyl, Hexinyl, Heptinyl, Octinyl, Noninyl oder Decinyl steht,
n für 0, 1 oder 2 steht.

6. Pyrazolylcarboxanilide der Formel (I) gemäß Anspruch 5, in welcher
G für Fluor, Chlor oder Methyl steht,
und R¹, R², R³ und n die in Anspruch 5 angegebenen Bedeutungen haben.

7. Pyrazolylcarboxanilide der Formel (I) gemäß einem oder mehreren der Ansprüche 1 bis 6 in welcher
R¹ für Methyl steht und
R² für Methyl steht.

8. Pyrazolylcarboxanilide der Formel (I) gemäß einem oder mehreren der Ansprüche 1 bis 2 in welcher
R³ für unsubstituiertes C₆-C₂₀-Alkyl steht.

9. Pyrazolylcarboxanilide der Formel (I) gemäß einem oder mehreren der Anspruche 1 bis 8, in welcher
n für 0 steht.

10. Pyrazolylcarboxanilide der Formel (I) gemäß einem oder mehreren der Ansprüche 1 bis 9, in welcher
R¹ für Methyl steht,
R² für Methyl steht,
R³ für 1,3-Dimethylbutyl steht,
n für 0 steht.

11. Verfahren zum Herstellen der Verbindungen der Formel (I) gemäß Anspruch 1, **dadurch gekennzeichnet, dass** man
a) Carbonsäure-Derivate der Formel (II) 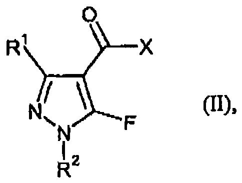 in welcher
R¹ und R² die in Anspruch 1 angegebenen Bedeutungen haben und
X für Halogen steht,
mit einem Anilinderivat der Formel (III) 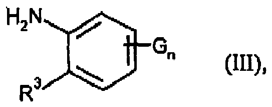 in welcher
G, R³ und n die in Anspruch 1 angegebenen Bedeutungen haben,
gegebenenfalls in Gegenwart eines Säurebindemittels und gegebenenfalls in Gegenwart eines Verdünnungsmittels umsetzt, oder
b) Pyrazolylcarboxanilide der Formel (Ia) 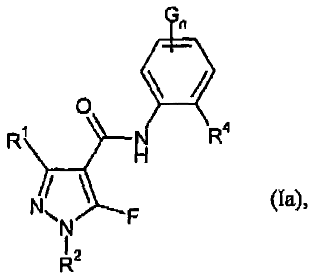 in welcher
R¹, R², G und n die in Anspruch 1 angegebenen Bedeutungen haben,
R⁴ für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart eines Katalysators hydriert, oder
c) Hydroxyalkylpyrazolylcarboxanilide der Formel (IV) 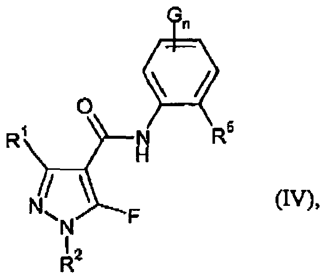 in welcher
R¹, R², G und n die in Anspruch 1 angegebenen Bedeutungen haben,
R⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart einer Säure dehydratisiert, oder
d) Hatogenpyrazolylcarboxanilide der Formel (V) 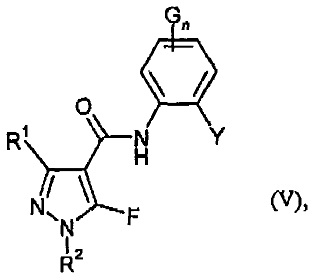 in welcher
R¹, R², G und n die in Anspruch 1 angegebenen Bedeutungen haben,
Y für Brom oder Iod steht,
mit einem Alkin der Formel (VI) 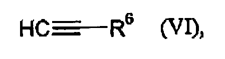 in welcher
R⁶ für gegebenenfalls einfach oder mehrfach, gleich oder verschieden durch Halogen und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
oder einem Alken der Formel (VII) 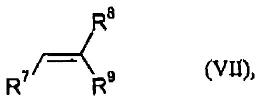 in welcher
R⁷, R⁸ und R⁹ unabhängig voneinander jeweils für Wasserstoff oder gegebenenfalls einfach oder mehrfach, gleich oder verschieden durch Halogen und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 20 nicht übersteigt,
gegebenenfalls in Gegenwart eines Verdünnungsmittels, gegebenenfalls in Gegenwart eines Säurebindemittels und in Gegenwart eines oder mehrerer Katalysatoren umsetzt, oder
e) Ketone der Formel (VIII) 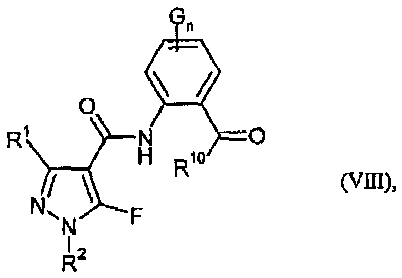 in welcher
R¹, R², G und n die in Anspruch 1 angegebenen Bedeutungen haben,
R¹⁰ für Wasserstoff oder gegebenenfalls einfach oder mehrfach, gleich oder verschieden durch Halogen und/oder C₃-C₆-cyclo, alkyl substituiertes C₁-C₁₈-Alkyl steht, wobei der Cycloalkylteil seinerseits gegebenenfalls durch Halogen und/oder C₁-C₄-Alkyl substituiert sein kann,
mit einer Phosphorverbindung der allgemeinen Formel (IX)
R¹¹―Px (IX),
in welcher
R¹¹ für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann und
Px für eine Gruppierung -P⁺(C₆H₅)₃ Cl⁻, -P⁺(C₆H₅)₃Br⁻, -P⁺(C₆H₅)₃ I⁻, -P(=O)(OCH₃)₃ oder -P(=O)(OC₂H₅)₃ steht,
gegebenenfalls in Gegenwart eines Verdünnungsmittels, umsetzt.

12. Mittel zum Bekämpfen unerwünschter Mikroorganismen, **gekennzeichnet durch** einen Gehalt an mindestens einem Pyrazolylcarboxanilid der Formel (I) gemäß Anspruch 1 neben Streckmitteln und/oder oberflächenaktiven Stoffen.

13. Verwendung von Pyrazolylcarboxaniliden der Formel (I) gemäß Anspruch 1 zum Bekämpfen unerwünschter Mikroorganismen.

14. Verfahren zum Bekämpfen unerwünschter Mikroorganismen, **dadurch gekennzeichnet, dass** man Pyrazolylcarboxanilide der Formel (I) gemäß Anspruch 1 auf die Mikroorganismen und/oder deren Lebensraum ausbringt.

15. Verfahren zum Herstellen von Mitteln zum Bekämpfen unerwünschter Mikroorganismen, **dadurch gekennzeichnet, dass** man Pyrazolylcarboxanilide der Formel (I) gemäß Anspruch 1 mit Streckmitteln und/oder oberflächenaktiven Stoffen vermischt,

16. Hydroxyalkylpyrazolylcarboxanilide der Formel (IV) 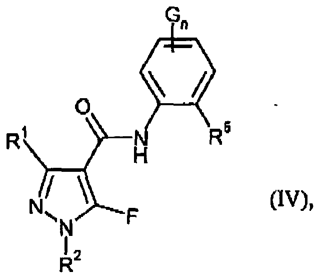 in welcher
R¹, R², G und n die in einem oder mehreren der Ansprüche 1 bis 7 angegebenen Bedeutungen haben und
R⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen, und/oder C₁-C₄-Alkyl substituiert sein kann,

17. Verfahren zum Herstellen von Hydroxyalkylpyrazolylcarboxaniliden der Formel (IV) gemäß Anspruch 13, **dadurch gekennzeichnet, dass** man
f) Carbonsäure-Derivate der Formel (II) 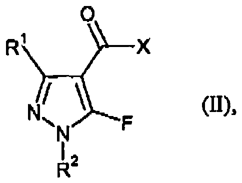 in welcher
R¹ und R² die in Anspruch 1 angegebenen Bedeutungen haben und
X für Halogen steht,
mit einem Hydroxyalkylanilinderivat der Formel (X) 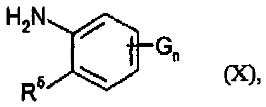 in welcher
R³, G und n die in Anspruch 1 angegebenen Bedeutungen haben,
R⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
gegebenenfalls in Gegenwart eines Säurebindemittels und gegebenenfalls in Gegenwart eines Verdünnungsmittels umsetzt.

18. Halogenpyrazolylcarboxanilide der Formel (V) 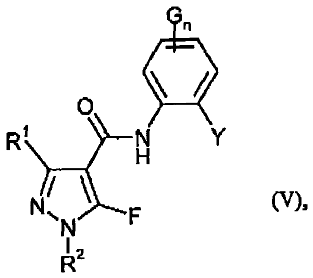 in welcher
R¹, R², G und n die in einem oder mehreren der Ansprüche 1 bis 7 angegebenen Bedeutungen haben und
Y für lod steht.

19. Verfahren zum Herstellen von Halogenpyrazolylcarboxaniliden der Formel (V) gemäß Anspruch 14, **dadurch gekennzeichnet, dass** man
g) Carbonsäure-Derivate der Formel (II) 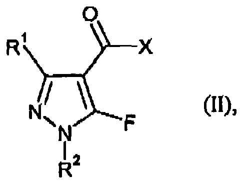 in welcher
R¹ und R² die in Anspruch 1 angegebenen Bedeutungen haben,
X für Halogen steht,
mit einem Halogenanilin der Formel (XI) 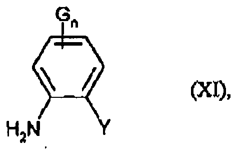 in welcher
G und n die in Anspruch 1 angegebenen Bedeutungen haben,
Y für Brom oder Iod steht,
gegebenenfalls in Gegenwart eines Säurebindemittels und gegebenenfalls in Gegenwart eines Verdilnnungsmittels umsetzt.

20. Ketone der Formel (VIII) 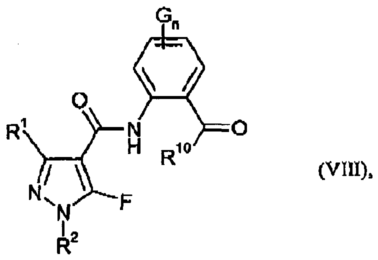 in welcher
R¹, R², G und n die in einem oder mehreren der Ansprüche 1 bis 7 angegebenen Bedeutungen haben und
R¹⁰ für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,

21. Verfahren zum Herstellen von Ketonen der Formel (VIII) gemäß Anspruch 17, **dadurch gekennzeichnet, dass** man
h) Carbonsäure-Derivate der Formel (II) 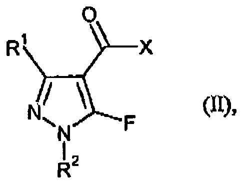 in welcher
R¹ und R² die in Anspruch 1 angegebenen Bedeutungen haben,
X für Halogen steht,
mit Ketoanilinen der Formel (XII) 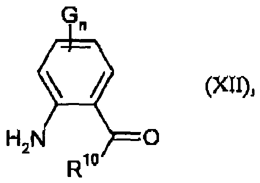 in welcher
G und n die in Anspruch 1 angegebenen Bedeutungen haben,
R¹⁰ für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
gegebenenfalls in Gegenwart eines Säurebindemittels und gegebenenfalls in Gegenwart eines Verdünnungsmittels umsetzt.

22. Verwendung der Pyrazolylcarboxanilide der Formel (I) gemäß Anspruch 1 zur Behandlung von Saatgut.

## Claims

1. Pyrazolylcarboxanilides of the formula (I) 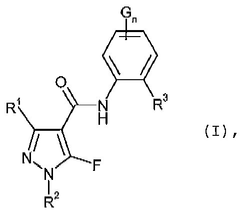 in which
R¹ represents hydrogen, cyano, halogen, nitro, C₁-C₄-alkyl, C₁-C₄-haloalkyl having 1 to 5 halogen atoms, C₃-C₆-cycloalkyl, C₁-C₄-alkoxy, C₁-C₄-haloalkoxy having 1 to 5 halogen atoms, C₁-C₄-alkylthio, C₁-C₄-haloalkylthio having 1 to 5 halogen atoms or aminocarbonyl-C₁-C₄-alkyl,
R² represents hydrogen, C₁-C₄-alkyl, C₁-C₆-haloalkyl having 1 to 5 halogen atoms, C₂-C₆-alkenyl, C₃-C₆-cycloalkyl, C₁-C₄-alkylthio-C₁-C₄-alkyl, C₁-C₄-haloalkylthio-C₁-C₄-alkyl having 1 to 5 halogen atoms, C₁-C₄-alkoxy-C₁-C₄-alkyl or C₁-C₄-haloalkoxy-C₁-C₄-alkyl having 1 to 5 halogen atoms,
G represents halogen or C₁-C₄-alkyl,
G furthermore represents C₅-C₆-alkyl,
R³ represents unsubstituted C₅-C₂₀-alkyl or represents C₁-C₂₀-alkyl which is mono- or polysubstituted by identical or different substituents from the group consisting of halogen and/or C₃-C₆-cycloalkyl or represents C₂-C₂₀-alkenyl or C₂-C₂₀-alkynyl, each of which is optionally mono- or polysubstituted by identical or different substituents from the group consisting of halogen and/or C₃-C₆-cycloalkyl, where the cycloalkyl moiety for its part may optionally be mono- or polysubstituted by identical or different substituents from the group consisting of halogen and/or C₁-C₄-alkyl and
n represents 0, 1 or 2.

2. Pyrazolylcarboxanilides of the formula (I) according to Claim 1, in which
G represents halogen or C₁-C₄-alkyl,
and R¹, R², R³ and n are as defined in Claim 1.

3. Pyrazolylcarboxanilides of the formula (I) according to Claim 1, in which
R¹ represents hydrogen, cyano, halogen, nitro, C₁-C₄-alkyl, C₁-C₄-haloalkyl having 1 to 5 fluorine, chlorine and/or bromine atoms, C₃-C₆-cycloalkyl, C₁-C₄-alkoxy, C₁-C₄-haloalkoxy having 1 to 5 fluorine, chlorine and/or bromine atoms, C₁-C₄-alkylthio, C₁-C₄-haloalkylthio having 1 to 5 fluorine, chlorine and/or bromine atoms or aminocarbonyl-C₁-C₄-alkyl,
R² represents hydrogen, C₁-C₄-alkyl, C₁-C₆-haloalkyl having 1 to 5 fluorine, chlorine and/or bromine atoms, C₂-C₆-alkenyl, C₃-C₆ cycloalkyl, C₁-C₄-alkylthio-C₁-C₄-alkyl, C₁-C₄-haloalkylthio-C₁-C₄-alkyl having 1 to 5 fluorine, chlorine and/or bromine atoms, C₁-C₄-alkoxy-C₁-C₄-alkyl or C₁-C₄-haloalkoxy-C₁-C₄-alkyl having 1 to 5 fluorine, chlorine and/or bromine atoms,
G represents halogen or C₁-C₄-alkyl,
G furthermore represents C₅-C₆-alkyl,
R³ represents unsubstituted C₆-C₁₂-alkyl or represents C₂-C₁₂-alkyl which is mono- to tetrasubstituted by identical or different substituents from the group consisting of fluorine, chlorine, bromine and/or C₃-C₆-cycloalkyl or represents C₂-C₁₂-alkenyl or C₂-C₁₂-alkynyl, each of which is optionally mono- to tetrasubstituted by identical or different substituents from the group consisting of fluorine, chlorine, bromine and/or C₃-C₆-cycloalkyl, where the moiety for its part may optionally be mono- to tetrasubstituted by identical or different substituents from the group consisting of halogen and/or C₁-C₄-alkyl,
n represents 0, 1 or 2.

4. Pyrazolylcarboxanilides of the formula (I) according to Claim 3, in which
G represents halogen or C₁-C₄-alkyl,
and R¹, R², R³ and n are as defined in Claim 3.

5. Pyrazolylcarboxanilides of the formula (I) according to Claim 1, in which
R¹ represents hydrogen, methyl, ethyl, n- or i-propyl, n-, i-, s- or t-butyl, trifluoromethyl or trifluoroethyl,
R² represents hydrogen, methyl, ethyl, n- or i-propyl, n-, i-, s- or t-butyl, trifluoromethyl or trifluoroethyl,
G represents fluorine, chlorine or methyl,
G furthermore represents ethyl or t-butyl,
G furthermore represents 2,4-dimethylbutyl,
R³ represents in each case straight-chain or branched ethyl, propyl, butyl, pentyl, hexyl, heptyl, octyl, nonyl, decyl, ethenyl, propenyl, butenyl, pentenyl, hexenyl, heptenyl, octenyl, nonenyl, decenyl, ethynyl, propynyl, butynyl, pentynyl, hexynyl, heptynyl, octynyl, nonynyl or decynyl, each of which may be attached at any position and each of which is optionally mono- to tetrasubstituted by identical or different substituents from the group consisting of fluorine, cyclopropyl, difluorocyclopropyl, cyclobutyl, cyclopentyl and/or cyclohexyl,
n represents 0, 1 or 2.

6. Pyrazolylcarboxanilides of the formula (I) according to Claim 5, in which
G represents fluorines, chlorine or methyl,
and R¹, R², R³ and n are as defined in Claim 5.

7. Pyrazolylcarboxanalides of the formula (I) according to one or more of Claims 1 to 6 in which
R¹ represents methyl and
R² represents methyl.

8. Pyrazolylcarboxanilides of the formula (I) according to one or more of Claims 1 to 2, in which
R³ represents unsubstituted C₂-C₂₀-alkyl.

9. Pyrazolylcarboxanilides of the formula (I) according to one or more of Claims 1 to 8, in which
n represents 0.

10. Pyrazolylcarboxanilides of the formula (I) according to one or more of Claims 1 to 9, in which
R¹ represents methyl,
R² represents methyl,
R³ represents 1,3-dimethylbutyl,
n represents 0.

11. Process for preparing compounds of the formula (I) according to Claim 1, **characterized in that**
a) carboxylic acid derivatives of the formula (II) 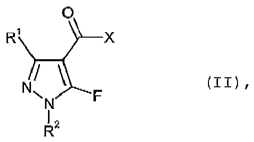 in which
R¹ and R² are as defined in Claim 1 and
X represents halogen,
are reacted with an aniline derivative of the formula (III) 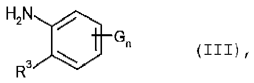 in which
G, R³ and n are as defined in Claim 1,
if appropriate in the presence of an acid binder and if appropriate in the presence of a diluent, or
b) pyrazolylcarboxanilides of the formula (Ia) 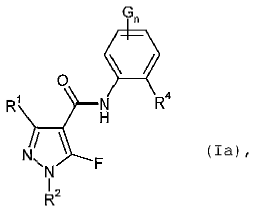 in which
R¹, R², G and n are as defined in Claim 1,
R⁴ represents C₂-C₂₀-alkenyl or C₂-C₂₀-alkynyl, each of which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl,
are hydrogenated, if appropriate in the presence of a diluent and if appropriate in the presence of a catalyst, or
c) hydroxyalkylpyrazolylcarboxanilides of the formula (IV) 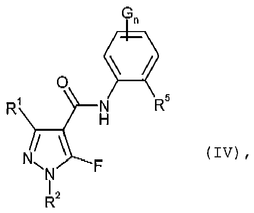 in which
R¹, R², G and n are as defined in Claim 1,
R⁵ represents C₂-C₂₀-hydroxyalkyl which is optionally additionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl,
is dehydrated, if appropriate in the presence of a diluent and if appropriate in the presence of an acid, or
d) halopyrazolylcarboxanilides of the formula (V) 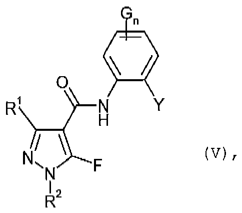 in which
R¹, R², G and n are as defined in Claim 1,
Y represents bromine or iodine,
are reacted with an alkyne of the formula (VI) 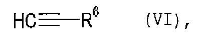 in which
R⁶ represents C₂-C₁₈-alkyl which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl,
or an alkene of the formula (VII) 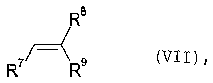 in which
R⁷, R⁸ and R⁹ independently of one another each represent hydrogen or alkyl which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl and where the total number of carbon atoms of the open-chain moiety does not exceed the number 20,
if appropriate in the presence of a diluent, if appropriate in the presence of an acid binder and in the presence of one or more catalysts, or
e) ketones of the formula (VIII) 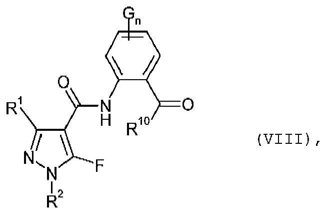 in which
R¹, R², and n are as defined in Claim 1,
R¹⁰ represents hydrogen or C₁-C₁₈-alkyl which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl,
are reacted with a phosphorus compound of the general formula (IX)
R¹¹―Px (IX),
in which
R¹¹ represents hydrogen or C₁-C₁₈-alkyl which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl and
Px represents a grouping -P⁺(C₆H₅)₃ Cl⁻, - P⁺(C₆H₅)₃ Br⁻, -P⁺(C₆H₅)₃ I⁻, -P(=O)(OCH₃)₃ or -P(=O)(OC₂H₅)₃,
if appropriate in the presence of a diluent.

12. Compositions for controlling undesirable microorganisms, **characterized in that** they comprise at least one pyrazolylcarboxanilide of the formula (I) according to Claim 1, in addition to extenders and/or surfactants.

13. Use of pyrazolylcarboxanilides of the formula (I) according to Claim 1 for controlling undesirable microorganisms.

14. Method for controlling undesirable microorganisms, **characterized in that** pyrazolylcarboxanilides of the formula (I) according to Claim 1 are applied to the microorganisms and/or their habitat.

15. Process for preparing compositions for controlling undesirable microorganisms, **characterized in that** pyrazolylcarboxanilides of the formula (I) according to Claim 1 are mixed with extenders and/or surfactants.

16. Hydroxyalkylpyrazolylcarboxanilides of the formula (IV) 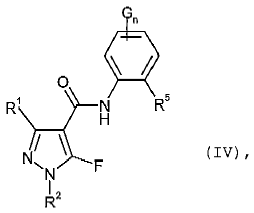 in which
R¹, R², G and n are as defined in one or more of Claims 1 to 7 and
R⁵ represents C₂-C₂₀-hydroxyalkyl which is optionally additionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl.

17. Process for preparing hydroxyalkylpyrazolylcarboxanilides of the formula (IV) according to Claim 13, **characterized in that**
f) carboxylic acid derivatives of the formula (II) 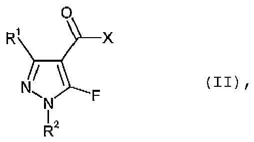 in which
R¹ and R² are as defined in Claim 1 and
X represents halogen,
are reacted with a hydroxyalkylaniline derivative of the formula (X) 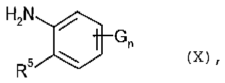 in which
R³, G and n are as defined in Claim 1,
R⁵ represents C₂-C₂₀-hydroxyalkyl which is optionally additionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl,
if appropriate in the presence of an acid binder and if appropriate in the presence of a diluent.

18. Halopyrazolylcarboxanilides of the formula (V) 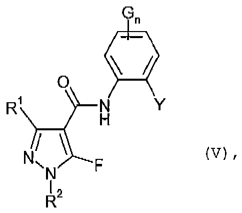 in which
R¹, R², G and n are as defined in one or more of Claims 1 to 7 and
Y represents iodine.

19. Process for preparing halopyrazolylcarboxanilides of the formula (V) according to Claim 14, **characterized in that**
g) carboxylic acid derivatives of the formula (II) 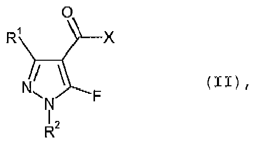 in which
R¹ and R² are as defined in Claim 1,
X represents halogen,
are reacted with a haloaniline of the formula (XI) 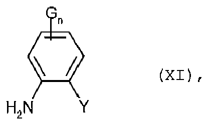 in which
G and n are as defined in Claim 1,
Y represents bromine or iodine,
if appropriate in the presence of an acid binder and if appropriate in the presence of a diluent.

20. Ketones of the formula (VIII) 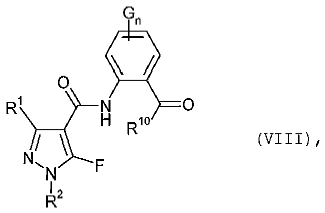 in which
R¹, R², G and n are as defined in one or more of Claims 1 to 7 and
R¹⁰ represents hydrogen or C₁-C₁₈-alkyl which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl.

21. Process for preparing ketones of the formula (VIII) according to Claim 17, **characterized in that**
h) carboxylic acid derivatives of the formula (II) 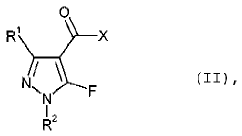 in which
R¹ and R² are as defined in Claim 1,
X represents halogen,
are reacted with ketoanilines of the formula (XII) 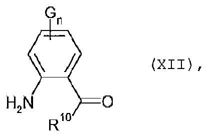 in which
G and n are as defined in Claim 1,
R¹⁰ represents hydrogen or C₁-C₁₈-alkyl which is optionally mono- or polysubstituted by identical or different substituents from the group consisting of halogen and/or C₃-C₆-cycloalkyl, where the cycloalkyl moiety for its part may optionally be substituted by halogen and/or C₁-C₄-alkyl,
if appropriate in the presence of an acid binder and if appropriate in the presence of a diluent.

22. Use of the pyrazolylcarboxanilides of the formula (I) according to Claim 1 for the treatment of seed.

## Revendications

1. Pyrazolylcarboxanilides de formule (I) 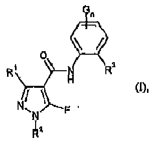 dans laquelle
R¹ représente hydrogène, cyano, halogène, nitro, C₁-C₄-alkyle, C₁-C₄-halogénoalkyle comprenant 1 à 5 atomes d'halogène, C₃-C₆-cycloalkyle, C₁-C₄-alcoxy, C₁-C₄-halogénoalcoxy comprenant 1 à 5 atomes d'halogène, C₁-C₄-alkylthio, C₁-C₄-halogénoalkylthio comprenant 1 à 5 halogènes ou aminocarbonyl-C₁-C₄-alkyle,
R² représente hydrogène, C₁-C₄-alkyle, C₁-C₆-halogénoalkyle comprenant 1 à 5 atomes d'halogène, C₂-C₆-alcényle, C₃-C₆-cycloalkyle, C₁-C₄-alkylthio-C₁-C₄-alkyle, C₁-C₄-halogénoalkylthio-C₁-C₄-alkyle-comprenant 1 à 5 atomes d'halogène, C₁-C₄-alcoxy-C₁-C₄-alkyle ou C₁-C₄-halogénoalcoxy-C₁-C₄-alkyle comprenant 1 à 5 atomes d'halogène,
G représente halogène ou C₁-C₄-alkyle,
G représente en outre C₅-C₆-alkyle,
R³ représente C₅-C₂₀-alkyle non substitué ou C₁-C₂₀-alkyle monosubstitué ou polysubstitué, de manière identique ou différente, par halogène et/ou C₃-C₆-cycloalkyle, ou C₂-C₂₀-alcényle ou C₂-C₂₀-alcynyle à chaque fois le cas échéant monosubstitué ou polysubstitué, de manière identique ou différente, par halogène et/ou C₃-C₆-cycloalkyle, la partie cycloalkyle pouvant à son tour le cas échéant être monosubstituée ou polysubstituée, de manière identique ou différente, par halogène et/ou C₁-C₄-alkyle et
n vaut 0, 1 ou 2.

2. Pyrazolylcarboxanilides de formule (I) selon la revendication 1, dans laquelle
G représente halogène ou C₁-C₄-alkyle, et
R¹, R², R³ et n présentent les significations indiquées dans la revendication 1.

3. Pyrazolylcarboxanilides de formule (I) selon la revendication 1, dans laquelle
R¹ représente hydrogène, cyano, halogène, nitro, C₁-C₄-alkyle, C₁-C₄-halogénoalkyle comprenant 1 à 5 atomes de fluor, de chlore et/ou de brome, C₃-C₆-cycloalkyle, C₁-C₄-alcoxy, C₁-C₄-halogénoalcoxy comprenant 1 à 5 atomes de fluor, de chlore et/ou de brome, C₁-C₄-alkylthio, C₁-C₄-halogénoalkylthio comprenant 1 à 5 atomes de fluor, de chlore et/ou de brome ou aminocarbonyl-C₁-C₄-alkyle,
R² représente hydrogène, C₁-C₄-alkyle, C₁-C₆-halogénoalkyle comprenant 1 à 5 atomes de fluor, de chlore et/ou de brome, C₂-C₆-alcényle, C₃-C₆-cycloalkyle, C₁-C₄-alkylthio-C₁-C₄-alkyle, C₁-C₄-halogénoalkylthio-C₁-C₄-alkyle comprenant 1 à 5 atomes de fluor, de chlore et/ou de brome, C₁-C₄-alcoxy-C₁-C₄-alkyle ou C₁-C₄-halogénoalcoxy-C₁-C₄-alkyle comprenant 1 à 5 atomes de fluor, de chlore et/ou de brome,
G représente halogène ou C₁-C₄-alkyle,
G représente en outre C₅-C₆-alkyle,
R³ représente C₆-C₁₂-alkyle non substitué ou C₂-C₁₂-alkyle monosubstitué à tétrasubstitué, de manière identique ou différente, par fluor, chlore, brome et/ou C₃-C₆-cycloalkyle, ou C₂-C₁₂-alcényle ou C₂-C₁₂-alcynyle à chaque fois le cas échéant monosubstitué à tétrasubstitué, de manière identique ou différente, par fluor, chlore, brome et/ou C₃-C₆-cycloalkyle, la partie cycloalkyle pouvant à son tour le cas échéant être monosubstituée à tétrasubstituée, de manière identique ou différente, par halogène et/ou C₁-C₄-alkyle
n vaut 0, 1 ou 2.

4. Pyrazolylcarboxanilides de formule (I) selon la revendication 3, dans laquelle
G représente halogène ou C₁-C₄-alkyle, et
R¹, R², R³ et n présentent les significations indiquées dans la revendication 3.

5. Pyrazolylcarboxanilides de formule (I) selon la revendication 1, dans laquelle
R¹ représente hydrogène, méthyle, éthyle, n-propyle ou i-propyle, n-butyle, i-butyle, s-butyle ou t-butyle, trifluorométhyle ou trifluoroéthyle,
R² représente hydrogène, méthyle, éthyle, n-propyle ou i-propyle, n-butyle, i-butyle, s-butyle ou t-butyle, trifluorométhyle ou trlfluoroéthyle,
G représente fluor, chlore ou méthyle,
G représente en outre éthyle ou t-butyle,
G représente en outre 2,4-diméthylbutyle,
R³ représente éthyle, propyle, butyle, pentyle, hexyle, heptyle, octyle, nonyle, décyle, éthényle, propényle, butényle, pentényle, hexényle, heptényle, octényle, nonényle, décényle, éthynyle, propynyle, butynyle, pentynyle, hexynyle, heptynyle, octynyle, nonynyle ou décynyle, à chaque fois linéaire ou ramifié, à chaque fois lié en un endroit quelconque, à chaque fois le cas échéant monosubstitué à tétrasubstitué, de manière identique ou différente, par fluor, cyclopropyle, difluorocyclopropyle, cyclobutyle, cyclopentyle et/ou cyclohexyle,
n vaut 0, 1 ou 2.

6. Pyrazolylcarboxanilides de formule (I) selon la revendication 5, dans laquelle
G représente fluor, chlore ou méthyle, et
R¹, R², R³ et n présentent les significations indiquées dans la revendication 5.

7. Pyrazolylcarboxanilides de formule (I) selon l'une ou plusieurs des revendications 1 à 6, dans laquelle
R¹ représente méthyle et
R² représente méthyle.

8. Pyrazolylcarboxanilides de formule (I) selon l'une ou plusieurs des revendications 1 à 2, dans laquelle
R³ représente C₆-C₂₀-alkyle non substitué.

9. Pyrazolylcarboxanilides de formule (I) selon l'une ou plusieurs des revendications 1 à 8, dans laquelle
n vaut 0.

10. Pyrazolylcarboxanilides de formule (I) selon l'une ou plusieurs des revendications 1 à 9, dans laquelle
R¹ représente méthyle,
R² représente méthyle,
R³ représente 1,3-diméthylbutyle,
n vaut 0.

11. Procédé pour la préparation des composés de formule (I) selon la revendication 1, **caractérisé en ce qu'**on
a) transforme des dérivés d'acide carboxylique de formule (II) 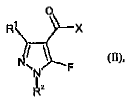 dans laquelle
R³ et R² présentent les significations indiquées dans la revendication 1 et
X représente halogène,
avec un dérivé d'aniline de formule (III) 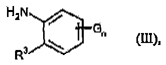 dans laquelle
G, R³ et n présentent les significations indiquées dans la revendication 1,
le cas échéant en présence d'un liant d'acide et le cas échéant en présence d'un diluant, ou
b) on hydrogène des pyrazolylcarboxanilides de formule (Ia) 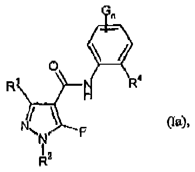 dans laquelle
R¹, R², G et n présentent les significations indiquées dans la revendication 1,
R⁴ représente C₂-C₂₀-alcényle ou C₂-C₂₀-alcynyle à chaque fois le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle,
le cas échéant en présence d'un diluant et le cas échéant en présence d'un catalyseur, ou
c) on déshydrate des hydroxyalkylpyrazolylcarboxanilides de formule (IV) 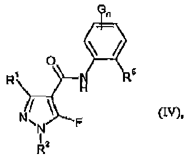 où
R¹, R², G et n présentent les significations indiquées dans la revendication 1,
R⁵ représente C₂-C₂₀-hydroxyalkyle, le cas échéant en plus monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle,
le cas échéant en présence d'un diluant et le cas échéant en présence d'un acide, ou
d) on transforme des halogénopyrazolylcarboxanilides de formule (V) 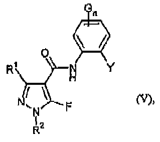 dans laquelle
R¹, R², G et n présentent les significations indiquées dans la revendication 1,
Y représente brome ou iode,
avec un alcyne de formule (VI) 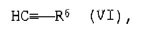 dans laquelle
R⁶ représente C₂-C₁₈-alkyle, le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle,
avec un alcène de formule (VII) 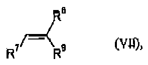 dans laquelle
R¹, R⁸ et R⁹ représentent, indépendamment l'un de l'autre, à chaque fois hydrogène ou alkyle le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle et le nombre total d'atomes de carbone de la partie de molécule à chaîne ouverte ne dépassant pas le nombre 20,
le cas échéant en présence d'un diluant, le cas échéant en présence d'un liant d'acide et en présence d'un ou de plusieurs catalyseurs,
e) on transforme des cétones de formule (VIII) 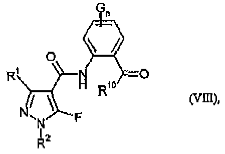 dans laquelle
R¹, R², G et n présentent les significations indiquées dans la revendication 1,
R¹⁰ représente hydrogène ou C₁-C₁₈alkyle, le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle,
avec un composé phosphoré de formule générale (IX)
R¹¹―Px (IX),
dans laquelle
R¹¹ représente hydrogène ou C₁-C₁₈-alkyle, le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle, et
Px représente un groupement -P⁺(C₆H₅)₃Cl⁻, -P⁺(C₆H₅)₃Br⁻, -P⁺(C₆H₅)₃I⁻, -P(=0) (OCH₃)₃ ou -P(=O) (OC₂H₅)₃,
le cas échéant en présence d'un diluant.

12. Agent pour lutter contre des microorganismes non souhaités, **caractérisé par** une teneur en au moins un pyrazolylcarboxanilide de formule (I) selon la revendication 1, outre des agents d'allongement et/ou des substances tensioactives.

13. Utilisation de pyrazolylcarboxanilides de formule (I) selon la revendication 1, pour lutter contre des microorganismes non souhaités.

14. Procédé pour lutter contre des microorganismes non souhaités, **caractérisé en ce qu'**on épand des pyrazolylcarboxanilides de formule (I) selon la revendication 1 sur les microorganismes et/ou leur espace de vie.

15. Procédé pour préparer des agents pour lutter contre des microorganismes non souhaités, **caractérisé en ce qu'**on mélange des pyrazolylcarboxanilides de formule (I) selon la revendication 1 avec des agents d'allongement et/ou des substances tensioactives.

16. Hydroxyalkylpyrazolylcarboxanilides de formule (IV) 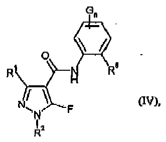 dans laquelle
R¹, R², G et n présentent les significations indiquées dans une ou plusieurs des revendications 1 à 7 et
R⁵ représente C₂-C₂₀-hydroxyalkyle, le cas échéant en plus monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle.

17. Procédé pour la préparation d'hydroxyalkylpyrazolylcarboxanilides de formule (IV) selon la revendication 13, **caractérisé en ce qu'**on
f) transforme des dérivés d'acide carboxylique de formule (II) 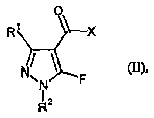 dans laquelle
R¹ et R² présentent les significations indiquées dans la revendication 1 et
X représente halogène,
avec un dérivé d'hydroxyalkylaniline de formule (X) 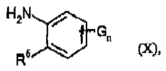 dans laquelle
R³, G et n présentent les significations indiquées dans la revendication 1,
R⁵ représente C₂-C₂₀-hydroxyalkyle, le cas échéant en plus monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle,
le cas échéant en présence d'un liant d'acide et le cas échéant en présence d'un diluant.

18. Halogénopyrazolylcarboxanilides de formule (V) 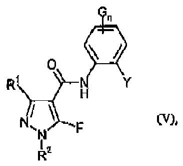 dans laquelle
R¹, R², G et n présentent les significations indiquées dans une ou plusieurs des revendications 1 à 7 et
Y représente iode.

19. Procédé pour la préparation d'halogénopyrazolylcarboxanilides de formule (V) selon la revendication 14, **caractérisé en ce qu'**on g) transforme des dérivés d'acide carboxylique de formule (II) 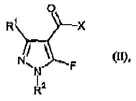 dans laquelle
R¹ et R² présentent les significations indiquées dans la revendication 1,
X représente halogène,
avec une halogénoaniline de formule (XI) 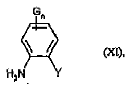 dans laquelle
G et n présentent les significations indiquées dans la revendication 1,
Y représente brome ou iode,
le cas échéant en présence d'un liant d'acide et le cas échéant en présence d'un diluant.

20. Cétones de formule (VIII) 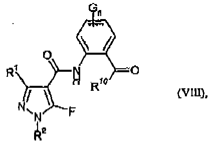 dans laquelle
R¹, R², G et n présentent les significations indiquées dans une ou plusieurs des revendications 1 à 7 et
R¹⁰ représente hydrogène ou C₁-C₁₈-alkyle, le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle.

21. Procédé pour la préparation de cétones de formule (VIII) selon la revendication 17, **caractérisé en ce qu'**on
h) transforme des dérivés d'acide carboxylique de formule (II) 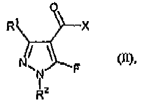 dans laquelle
R¹ et R² présentent les significations indiquées dans la revendication 1,
X représente halogène,
avec des cétoanilines de formule (XII) 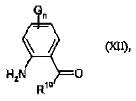 dans laquelle
G et n présentent les significations indiquées dans la revendication 1,
R¹⁰ représente hydrogène ou C₁-C₁₈-alkyle, le cas échéant monosubstitué ou polysubstitué, de manière identique ou différente, par halogène et/ou C₃-C₆-cycloalkyle, la partie cycloalkyle pouvant à son tour le cas échéant être substituée par halogène et/ou C₁-C₄-alkyle,
le cas échéant en présence d'un liant d'acide et le cas échéant en présence d'un diluant.

22. Utilisation des pyrazolyloarboxanilides de formule (I) selon la revendication 1 pour le traitement de semences.
